# EUROPEAN PATENT APPLICATION

(11) **EP 1 338 655 A1**
(43) Date of publication of application: **27.08.2003**
(21) Application number: 01972658.7
(22) Date of filing: 02.10.2001
(51) Int. Cl.: C12Q 1/02, C12Q 1/48, C12N 15/09, C07K 16/18, C07K 14/47, A61K 39/395, A61P 43/00, A61P 35/00, A61P 9/00, A61K 45/00, G01N 33/15, G01N 33/50

(54) **SUBSTANCE INHIBITING THE BINDING OF SIGNAL TRANSDUCING MOLECULE TO KDR/FLK-1 PHOSPHORYLATED AT TYROSINE AT THE 1175-POSITION AND METHOD OF USING THE SAME**

(30) Priority: 03.10.2000 JP 2000000303
(71) Applicant: Kyowa Hakko Kogyo Co., Ltd, Tokyo 100-8185 (JP); Shibuya, Masabumi, Kawaguchi-shi, Saitama 333-0866 (JP)
(72) Inventor: SHIBUYA, Masabumi, Kawaguchi-shi, Saitama 333-0866 (JP); TAKAHASHI, Tomoko, Narashino-shi, Chiba 275-0021 (JP); Furuya, Akiko Tokyo Res. Lab. kyowa Hakko Kog.Co., Machida-shi, Tokyo 194-0023 (JP); Shitara, Kenya Tokyo Res. Lab. Kyowa Hakko Kog.Co, Machida-shi, Tokyo 194-0023 (JP)
(74) Representative: VOSSIUS & PARTNER
(86) International application number: JP0108684
(87) International publication number: WO02029090

(57) **Abstract**

The present invention provides a substance which inhibits binding of a signal transduction molecule to 1175-tyrosine phosphorylated KDR/Flk-1 and inhibits cell growth by VEGF. Also, the present invention provides a method for inhibiting KDR/Flk-1 signal transduction, a method for inhibiting cell growth, a method for inhibiting angiogenesis, a method for screening a cell growth inhibitor, a method for screening an angiogenesis inhibitor, a method for screening a substance which inhibits KDR/Flk-1 signal transduction, a method for determining whether or not a tested substance can inhibit KDR/Flk-1 signal transduction, a method for detecting angiogenesis in tissue and a method for screening a substance which inhibits 1175-tyrosine phosphorylated. KDR/Flk-1, in which each method comprises using the substance.

## Description

### TECHNICAL FIELD

The present invention relates to a substance which inhibits binding of a signal transduction molecule to 1175-tyrosine phosphorylated KDR/Flk-1. Also, the present invention relates to a method for inhibiting KDR/Flk-1 signal transduction, a method for inhibiting cell growth, a method for inhibiting angiogenesis, a method for screening a cell growth inhibitor, a method for screening an angiogenesis inhibitor, a method for screening a substance which inhibits KDR/Flk-1 signal transduction, a method for determining whether or not a tested substance inhibits KDR/Flk-1 signal transduction, a method for detecting angiogenesis in a tissue and a method for screening a substance which inhibits phosphorylation of 1175-tyrosine of KDR/Flk-1, in which each method comprises using the substance.

### BACKGROUND ART

Angiogenesis plays an important role in the individual development and construction of tissues in vertebrates and is closely involved in the formation of the corpus luteum during the sexual cycle, transient proliferation of the uterine endometrium and formation of the placenta in mature individuals (females). With regard to pathological states, angiogenesis is involved in the proliferation or metastasis of solid tumors and formation or acceleration of morbidity in diabetic retinopathy and rheumatoid arthritis (*J. Biol. Chem*., 267, 10931 (1992)). Angiogenesis occurs by the secretion of an angiogenesis factor and involves the process of a tube formation and producing a new blood vessel. During this process, the basement membrane and interstitum are destroyed by a protease secreted from endothelial cells of an existing blood vessel around the secreted angiogenesis factor, followed by subsequent migration and proliferation of vascular endothelial cells (*J. Biol. Chem*., 267, 10931 (1992)). Factors which induce angiogenesis include vascular permeability factor (hereinafter referred to as "VPF") and vascular endothelial growth factor (hereinafter referred to as "VEGF") (hereinafter referred to as "VPF/VEGF"). These factors are considered the most important factors in pathological and non-pathological angiogenesis (*Advances in* *Cancer Research,* 67, 281 (1995)). VPF/VEGF is a protein having a molecular weight of about 40,000 constituted by homodimers, which had been reported to be independent molecules as vascular permeability factor (VPF) in 1983 *(Science,* 219, 983 (1983)) and as vascular endothelial growth factor (VEGF) in 1989 (*Biochem. Biophys. Res. Comm.,* 161, 851 (1989)), but it has been revealed as the results of cDNA cloning that they are the same substance (*Science,* 246, 1306 (1989); *Science,* 246, 1309 (1989)) (hereinafter, the term "VPF/VEGF" is referred to as "VEGF"). Beyond the activity of VEGF upon vascular endothelial cells described above, VEGF has also been shown to have a growth enhancing activity (*Biochem. Biophys. Res. Comm.,* 161, 851 (1989)), a migration enhancing activity (*J. Immunology,* 152, 4149 (1994)), a metalloprotease secretion enhancing activity (*J. Cell Physiol*., 153, 557 (1992)), a urokinase and tPA secretion enhancing activity (*Biochem. Biophys. Res. Comm*., 181, 902 (1991)) and the like. Furthermore, VEGF has been shown to have an angiogenesis enhancing activity (*Circulation*, 92 suppi II, 365 (1995)), a vascular permeability enhancing activity (*Science*, 219, 983 (1983)) and the like as its in vivo activities. It has been reported that VEGF is a growth factor having extremely high specificity for vascular endothelial cells (*Biochem. Biophys. Res. Comm.,* 161, 851 (1989)) and that four proteins having different molecular weight are present due to alternative splicing of mRNA (*J. Biol. Chem*., 67, 26031 (1991)).

Among diseases accompanied by angiogenesis, it has been reported that VEGF plays an important role in the proliferation or metastasis of solid tumors and formation of morbid states of diabetic retinopathy and chronic rheumatoid arthritis. With regard to solid tumors, production of VEGF in a number of human tumor tissues has been reported, such as in renal carcinoma (*Cancer Research*, 54, 4233 (1994)), breast cancer (*Human Pathology*, 26, 86 (1995)), brain tumor (*J. Clinical Investigation,* 91, 153 (1993)), gastrointestinal cancer (*Cancer Research*, 53, 4727 (1993)), ovarian cancer (*Cancer Research*, 54, 276 (1994)) and the like. Also, results of a study on the correlation between VEGF expression quantity in tumors and survival ratio of patients with breast cancer have revealed that tumor angiogenesis is more active in tumors expressing high levels of VEGF than low VEGF expression tumors and that the survival ratio is lower in breast cancer patients having high VEGF expression tumors than breast cancer patients having low VEGF expression tumors (*Japanese J*. *Cancer Research*, 85, 1045 (1994)). It has also been reported that an anti-VEGF monoclonal antibody inhibited tumor growth in a test system using a xenograft model in which a human tumor was transferred into nude mice by subcutaneous transplantation (*Nature*, 362, 841 (1993)). Also, it has been reported that, in a metastatic cancer model of a human tumor in nude mice, an anti-VEGF monoclonal antibody inhibited metastasis of the tumor (*Cancer Research*, 56, 921 (1996)). Additionally, since a high concentration of VEGF was detected in human carcinomatous pleural perfusions and ascites, the possibility that VEGF is a major factor involved in the retention of pleural perfusions and ascites has been suggested (*Biochimica et Biophysica Acta*, 1221, 211 (1994)),

In diabetic retinopathy, abnormal angiogenesis causes retinal detachment and hemorrhage of the vitreous body, resulting in blindness, and it has been reported that angiogenesis in diabetic retinopathy and the expression level of VEGF in the patient's eyeballs are positively correlative (*New England J. Medicine,* 331, 1480 (1994)). Also, it has been reported that angiogenesis in a monkey retinopathy model is inhibited when the VEGF activity is inhibited by the intraocular administration of an anti-VEGF neutralizing monoclonal antibody (*Arch. Ophthalmol*., 114, 66 (1996)).

Progress in the morbid states of rheumatoid arthritis (destruction of bone and cartilage) is accompanied by angiogenesis, and it has been reported that a high concentration of VEGF is contained in the synovial fluid of patients with rheumatoid arthritis and that macrophages in joints of patients with rheumatoid arthritis produce VEGF (*Journal of Immunology*, 152, 4149 (1994); *J. Experimental Medicine*, 180, 341 (1994)).

As receptors of VEGF, Flt-1 (fms-like tyrosine kinase) (Shibuya M *et al*., *Oncogene*, 5, 519 (1990), de Vries C *et al.*, *Science*, 255, 989 (1992)) and KDR (kinase insert domain-containing receptor) (WO 92/14748, Terman BI *et al., Biochem. Biophys*. *Res. Commun*., 187, 1579 (1992)) have bee reported. Since KDR has been discovered as Flk-1 (fetal liver kinase-1) in mouse (W. Matthews *et al., Proc. Natl. Acad. Sci. USA*, 88, 9026 (1991), WO 94/11499, Millauer B *et al.*, *Cell*, 72, 835 (1993)), they are generally referred to as KDR/Flk-1 herein.

Both of Flt-1 and KDR/Flk-1 are membrane proteins of 180 to 200 kDa in molecular weight belonging to the receptor tyrosine kinase family which have 7 immunoglobulin-like domains as an extracellular domain and a tyrosine kinase domain as an intracellular domain. VEGF specifically binds to Flt-1 and KDR/Flk-1 at K_{D} values of 20 pmol/l and 75 pmol/l, respectively. It has been reported that Flt-1 and KDR/Flk-1 are specifically expressed in vascular endothelial cells (Quinn TP *et al., Proc. Natl. Acad. Sci. USA*, 90, 7533 (1993), Kendall RL *et al., Proc. Natl. Acad. Sci. USA*, 90, 8915 (1993)).

It has been reported that expression of KDR/Flk-1 at mRNA level is increased in tumor vascular endothelial cells of a human brain tumor tissue (Hatva E *et al., Am. J. Pathol*., 146, 368 (1995)) and in tumor vascular endothelial cells of a human digestive organ tumor tissue (Brown LF *et al.*, *Cancer Res*., 53, 4727 (1993)), in comparison with vascular endothelial cells of normal tissues. These results strongly suggest that the VEGF-KDR/Flk-1 plays an important role in tumor angiogenesis. Furthermore, since it has been reported that expression of mRNA for KDR/Flk-1 was observed by *in situ* hybridization in vascular endothelial cells of joints of rheumatoid arthritis patients (Fava RA *et al., J*. *Exp. Med*., 180, 341 (1994)), importance of the VEGF-KDR/Flk-1 in rheumatoid arthritis is suggested.

With regard to functions of KDR/Flk-1, it has been reported that since pig artery vascular endothelial cells grow and migrate by reacting with VEGF when KDR/Flk-1 is expressed in the cells, KDR/Flk-1 relates to the growth and migration of vascular endothelial cells among various activities of VEGF (Waltenberger J *et al., J. Biol*. *Chem*., 269, 26988 (1994)). Also, it has been reported that since growth and blood vessel formation were not found and blood island of yolk sac was not formed in knockout mice in which the KDR/Flk-1 gene had been destroyed, and they died at a fetal stage of days 8.5 to 9.5 after development, KDR/Flk-1 relates to the growth and differentiation of vascular endothelial cells in an animal (Shalaby F *et al., Nature*, 376, 62 (1995)).

On the other hand, since knockout mice of Flt-1 gene die at the same fetal stage due to excess growth of endothelial cells and inability to form a normal structure of vascular endothelium (Fong G-H *et al.*, *Nature*, 376, 66 (1995)), it is considered that Flt-1 relates to the formation of a normal vascular endothelium structure as a negative controlling factor to inhibit growth of endothelial cells and that KDR/Flk-1 and Flt-1 play different roles of exerting the activity of VEGF.

It has been reported that a monoclonal antibody against the extracellular domain of KDR/Flk-1, which can inhibit binding of VEGF and KDR/Flk-1, inhibits KDR/Flk-1 signal transduction in vascular endothelial cells *in vitro* and thereby inhibits their growth (Rockwell P *et al., Mol. Cell. Differ*., 3, 91 (1995)), and that this antibody inhibits angiogenesis, growth and metastasis of various cancer cells transplanted into mice (Prewett M *et al., Cancer Res*., 59, 5209 (1999)). Also, it has been reported that an inhibitor SU5416 specific for the tyrosine kinase of KDR/Flk-1 inhibits angiogenesis, growth or metastasis of various cancer cells transplanted into mice (Annie T *et al., Cancer Res.,* 59, 99 (1999), Shaheen RM *et al., Cancer Res.,* 59, 5412 (1999)).

It is considered that signal transduction of a tyrosine kinase type receptor generally starts when its own tyrosine kinase is activated by binding to a ligand and its own and other molecule's tyrosine residues are phosphorylated. In this case, it is considered that other protein molecule binds to an auto-phosphorylated tyrosine of the receptor via SH2 domain, and that the molecule binds to other molecule or cause an enzyme reaction to thereby cause signal transduction. In the KDR/Flk-1, it has also been reported that its own tyrosine residue is phosphorylated by binding to VEGF in vascular endothelial cells (J. Wattenberger *et al*., *J. Biol. Chem.,* 269, 26988 (1994)). With regard to the position of the auto-phosphorylated tyrosine, it has so far been reported that 951-, 996-, 1054- and 1059-tyrosine residues in KDR/Flk-1 expressed in *Escherichia coli* are auto-phosphorylated (Dougher-Vermazen *et al., Biochem Biophys*. *Res. Commun*., 205, 728 (1994)) and that 801- and 1175-tyrosine residues in a sequence analogous to the consensus sequence to which phospholipase C-γ (hereinafter referred to as "PLC-γ") binds are phosphorylated in a model using yeast (Cunningham SA *et al*., *Biochem. Biophys. Res. Commun*., 240, 635 (1997)). It has been reported that the auto-phosphorylation sites of KDR/Flk-1 expressed in animal cells are mainly the 1175-and 1214-tyrosine residucs and the 801-tyrosine is hardly auto-phosphorylated (Takatiashi *et al., 5th Meeting of The Society of Vascular Medicine,* 2000). Also, it has been found by a yeast two hybrid system that an adapter protein Sck binds to the 1175-tyrosine of KDR/Flk-1 via its SH2 domain (Igarashi K *et al., Biochem. Biophys. Res. Commun*., 251, 72 (1998)).

With regard to the KDR/Flk-1 signal transduction reaching the growth of vascular endothelial cells, it is considered that Ras and PI3 kinase hardly relates thereto, which is different from other tyrosine kinase type receptors, and a PLC-γ-PKC-MAP kinase system mainly relates thereto, in which KDR/Flk-1 is activated and auto-phosphorylated by VEGF stimulation, PLC-γ binds to the KDR/Flk-1 and is then activated by phosphorylation, PKC is subsequently activated, and as a result MAP kinase is activated and DNA synthesis is induced (Takahashi T & Shibuya M, *Oncogene*, 14, 2079 (1997), Takahashi T *et al., Oncogene*, 18, 2221 (1999)). It has been reported that phosphorylation of 1175-tyrosine participates in the phosphorylation of PLC-γ and activation of MAP kinase by expressing a mutant of KDR/Flk-1, in which 1175- and 1214-tyrosine residues are replaced by phenylalanine residues, in a cell line derived from endothelial cells (Takahashi *et al., The 58th Meeting of The Japanese Cancer Association*, 1999).

Also, it has been reported that the 1175-tyrosine is phosphorylated VEGF-dependently in endothelial cells. In addition, it has also been reported that when a polyclonal antibody against the phosphorylated 1175-tyrosine was prepared, the antibody specifically recognized KDR/Flk-1 in which the 1175-tyrosine was phosphorylated (Takahashi *et al., 5th Meeting of The Society of Vascular Medicine,* 2000). However, the relationship between the antibody against the phosphorylatcd 1175-tyrosine and inhibition of cell growth is unknown.

### DISCLOSURE OF THE INVENTION

When signal transduction of the human VEGF receptor KDR can be inhibited, it will be useful in treating diseases whose morbid states progress by abnormal angiogenesis, such as solid tumor growth, metastasis formation, arthritis in rheumatoid arthritis, diabetic retinopathy, retinopathy of prematurity, psoriasis and the like in human.

An object of the present invention is to obtain a substance which blocks intracellular KDR/Flk-1 signal transduction and inhibits cell growth by VEGF to provide application methods of the substance.

An antibody against KDR/Flk-1 whose 1175-tyrosine, an auto-phosphorylation site important for the KDR/Flk-1 signal transduction, was phosphorylated was prepared. Thereafter, the inventors found that not only the antibody can specifically detect 1175-tyrosine phosphorylated KDR/Flk-1 but also, when the antibody is injected into endothelial cells, it can inhibit KDR/Flk-1 signal transduction and thereby inhibit VEGF-dependent cell growth.

The present invention relates to the following (1) to (41).
(1) A method for inhibiting KDR/Flk-1 signal transduction, which comprises using a substance which inhibits binding of a signal transduction molecule to 1175-tyrosine phosphorylated KDR/Flk-1.
(2) A method for inhibiting cell growth, which comprises using a substance which inhibits binding of a signal transduction molecule to 1175-thyroxin phosphorylated KDR/Flk-1.
(3) A method for inhibiting angiogenesis, which comprises using a substance which inhibits binding of a signal transduction molecule to 1175-tyrosine phosphorylated KDR/Flk-1.
(4) A method for screening a cell growth inhibitor, which comprises using a substance which inhibits binding of a signal transduction molecule to 1175-tyrosine phosphorylated KDR/Flk-1.
(5) A method for screening an angiogenesis inhibitor, which comprises using a substance which inhibits binding of a signal transduction molecule to 1175-tyrosine phosphorylated KDR/Flk-1.
(6) A method for screening a substance which inhibits KDR/Flk-1 signal transduction, which comprises using a substance which inhibits binding of a signal transduction molecule to 1175-tyrosine phosphorylated KDR/Flk-1.
(7) A method for determining whether or not a tested substance can inhibit KDR/Flk-1 signal transduction, which comprises using a substance which inhibits binding of a signal transduction molecule to 1175-tyrosine phosphorylated KDR/Flk-1.
(8) A method for detecting angiogenesis in tissue, which comprises using a substance which inhibits binding of a signal transduction molecule to 1175-tyrosine phosphorylated KDR/Flk-1.
(9) A method for screening a substance which inhibits 1175-tyrosine phosphorylated KDR/Flk-1, which comprises using a substance which inhibits binding of a signal transduction molecule to 1175-tyrosine phosphorylated KDR/Flk-1.
(10) The method according to any one of (1) to (9), wherein the signal transduction molecule is phospholipase C-γ (PLC-γ).
(11) The method according to any one of (1) to (9), wherein the substance which inhibits binding of a signal transduction molecule to 1175-tyrosine phosphorylated KDR/Flk-1 is an antibody which specifically recognizes 1175-tyrosine phosphorylated KDR/Flk-1.
(12) The method according to (11), wherein the antibody which specifically recognizes 1175-tyrosine phosphorylated KDR/Flk-1 is an antibody which binds to the 1175-tyrosine phosphorylated KDR/Flk-1 and inhibits phosphorylation of PLC-γ.
(13) The method according to (11) or (12), wherein the antibody is a monoclonal antibody or the antibody fragment thereof.
(14) A method for inhibiting KDR/Flk-1 signal transduction, which comprises using a substance which inhibits phosphorylation of 1175-tyrosine of KDR/Flk-1.
(15) A method for inhibiting cell growth, which comprises using a substance which inhibits phosphorylation of 1175-tyrosine of KDR/Flk-1.
(16) A method for inhibiting angiogenesis, which comprises using a substance which inhibits phosphorylation of 1175-tyrosine of KDR/Flk-1.
(17) A method for determining whether or not a tested substance can inhibit KDR/Flk-1 signal transduction, which comprises using a substance which inhibits phosphorylation of 1175-tyrosine of KDR/Flk-1.
(18) A method for detecting angiogenesis in tissue, which comprises using a substance which inhibits phosphorylation of 1175-tyrosine of KDR/Flk-1.
(19) An agent comprising, as an active ingredient, a substance which inhibits binding of a signal transduction molecule to 1175-tyrosine phosphorylated KDR/Flk-1.
(20) The agent according to (19), which is a tyrosine phosphorylation inhibitor.
(21) The agent according to (19), which is a cell growth inhibitor.
(22) The agent according to (19), which is an angiogenesis inhibitor.
(23) The agent according to any one of (19) to (22), wherein the substanc which inhibits binding of a signal transduction molecule to 1175-tyrosin phosphorylated KDR/Flk-1 is an antibody which specifically recognizes 1175-tyrosin phosphorylated KDR/Flk-1.
(24) The agent according to (23), wherein the antibody which specificall recognizes 1175-tyrosine phosphorylated KDR/Flk-1 is an antibody which binds to the 1175-tyrosine phosphorylated KDR/Flk-1 and inhibits phosphorylation phospholipase C-γ (PLC-γ).
(25) The agent according to (23) or (24), wherein the antibody is monoclonal antibody or an antibody fragment thereof.
(26) A tyrosine phosphorylation inhibitor of KDR/Flk-1, comprising, as a active ingredient, a substance which inhibits phosphorylation of 1175-tyrosine of KDR/Flk-1.
(27) A cell growth inhibitor, comprising as an active ingredient, a substance which inhibits phosphorylation of 1175-tyrosine of KDR/Flk-1.
(28) An angiogenesis inhibitor, comprising, as an active ingredient, substance which inhibits phosphorylation of 1175-tyrosine of KDR/Flk-1.
(29) A compound which is obtained by the method according to any one of (4) to (6) and (9).
(30) A monoclonal antibody or the antibody fragment thereof, which recognizes 1175-tyrosine phosphorylated KDR/Flk-1.
(31) The monoclonal antibody or the antibody fragment thereof accordin to (30), which is a monoclonal antibody or the antibody fragment thereof which binds to 1175-tyrosine phosphorylated KDR/Flk-1 and inhibits binding of a signal transduction molecule to the phosphorylated KDR/Flk-1.
(32) The monoclonal antibody or the antibody fragment thereof according to (30) or (31), which is a monoclonal antibody or the antibody fragment thereof which binds to 1175-tyrosine phosphorylated KDR/Flk-1 and inhibits phosphorylation of phospholipase C-γ (PLC-γ).
(33) The monoclonal antibody or the antibody fragment thereof according to any one of (30) to (32), which is a monoclonal antibody which is produced by a hybridoma KM3035 (FERM BP-7729) or the antibody fragment thereof.
(34) The antibody fragment according to any one of (30) to (33), wherein the antibody fragment is an antibody fragment selected from Fab, Fab', F(ab')₂, a single chain antibody (scFv), a dimerized variable region (V region) fragment (diabody), a disulfide stabilized V region fragment (dsFv) and a peptide comprising a complementarity determining region (CDR).
(35) A monoclonal antibody or the antibody fragment thereof, in which the monoclonal antibody or the antibody fragment thereof according to any one of (30) to (34) is chemically or genetically conjugated with a radioisotope, a protein or an agent.
(36) A DNA which encodes the monoclonal antibody or the antibody fragment thereof according to any one of (30) to (35).
(37) A recombinant vector comprising the DNA according to (36).
(38) A transformant comprising a host cell into which the recombinant vector according to (37) is introduced.

Examples of the substance which blocks intracellular KDR/Flk-1 signal transduction and inhibits cell growth by VEGF include a substance which inhibits binding of a signal transduction molecule to 1175-tyrosine phosphorylated KDR/Flk-1 and a substance which specifically inhibits 1175-tyrosine phosphorylation of KDR/Flk-1.

Examples of the substance which inhibits binding of a signal transduction molecule to 1175-tyrosine phosphorylated KDR/Flk-1 include an antibody or the antibody fragment thereof which specifically recognizes 1175-tyrosine phosphorylated KDR/Flk-1 and a compound that inhibits binding of a signal transduction molecule to 1175-tyrosine phosphorylated KDR/Flk-1.

As the antibody or an antibody fragment thereof of the present invention which specifically recognizes 1175-tyrosine phosphorylated KDR/Flk-1, any antibody having such a specificity that it specifically recognizes 1175-tyrosine phosphorylated KDR/Flk-1 and thereby binds thereto but does not bind to KDR/Flk-1 in which 1175-tyrosine is not phosphorylated (hereinafter also referred to as "anti-KDR antibody specific for 1175-tyrosine phosphorylation" or "antibody of the present invention") or the antibody fragment thereof can be used. But, an antibody or the antibody fragment thereof which inhibits binding of KDR/Flk-1 to PLC-γ is preferable.

Examples of the antibody of the present invention include a polyclonal antibody and a monoclonal antibody. A monoclonal antibody is preferable.

Examples of the monoclonal antibody include an antibody produced by a hybridoma and a recombinant antibody produced by a transformant transformed with an expression vector comprising an antibody gene.

Examples of the recombinant antibody include an antibody which is genetically produced, such as a humanized antibody, a human antibody, an antibody fragment and the like. A recombinant antibody having characteristics of a monoclonal antibody, low antigenicity and prolonged serum half-life is preferably used.

Examples of the humanized antibody include a human chimeric antibody and a human CDR-grafted antibody.

The antibody fragment includes fragments which specifically react with 1175-tyrosine phosphorylated KDR/Flk-1 such as Fab (abbreviation of fragment of antigen binding), Fab', F(ab')₂, a single chain antibody (single chain Fv; hereinafter referred to as "scFv") *(Science,* 242, 423 (1988)), a dimerizated variable region (also referred to as "V region") fragment (hereinafter referred to as "diabody") *(Nature Biotechnol*., 15, 629 (1997)) and a disulfide stabilized V region fragment (hereinafter referred to as "dsFv") (*Molecular Immunol*., 32, 249 (1995)).

Also, the antibody fragment includes a peptide comprising an amino acid sequence of a complementary determining region (hereinafter referred to as "CDR") of an antibody V region heavy chain (also referred to as "H chain") (hereinafter, the antibody variable heavy chain is also referred to as "VH") or an antibody V region light chain (also referred to as "L chain") (hereinafter, the antibody variable light chain is also referred to as "VL") of the above antibody (hereinafter referred to as "peptide comprising CDR") (*J. Biol*. *Chem*., 271, 2966 (1996)).

A human chimeric antibody is an antibody comprising an antibody variable region heavy chain and an antibody variable region light chain derived from a non-human animal, a constant region heavy chain (hereinafter referred to as "CH") derived from a human antibody and a constant region light chain (hereinafter referred to as "CL") derived from a human antibody.

The human chimeric antibody of the present invention can be produced by obtaining cDNAs encoding VH and VL from a hybridoma which produces an anti-KDR monoclonal antibody specific for 1175-tyrosine phosphorylation, inserting the cDNAs into an animal cell expression vector having genes encoding a human antibody CH and a human antibody CL to construct a human chimeric antibody expression vector, and introducing the vector into an animal cell to express the antibody.

The structure of the human chimeric antibody of the present invention may belong to any immunoglobulin (Ig) class. The C region of the IgG class is preferable, and the C region of immunoglobulin such as IgG1, IgG2, IgG3, IgG4 or the like belonging to the IgG class is more preferable.

A human CDR-grafted antibody is an antibody in which CDRs of VH and VL of a human antibody are substituted with CDRs, respectively, of an antibody derived from a non-human animal.

The human CDR-grafted antibody of the present invention can be produced by constructing cDNAs encoding V regions in which CDRs of VH and VL of a human antibody have been substituted with CDRs of VH and VL, respectively, of an antibody derived from a non-human animal which specifically reacts with 1175-tyrosine phosphorylated KDR/Flk-1, inserting the cDNAs into an animal cell expression vector having genes encoding CH of a human antibody and CL of a human antibody to construct a human CDR-grafted antibody expression vector, and introducing the expression vector into an animal cell to express the antibody.

The structure of the C region of the human CDR-grafted antibody of the present invention may belong to any immunoglobulin (Ig) class. The C region of the IgG class is preferable, and the C region of immunoglobulin such as IgG1, IgG2, IgG3, IgG4 or the like belonging to the IgG class is more preferable.

An Fab is a fragment having about 50,000 molecular weight and an antigen binding activity, which is constituted by about half of the N-terminal side of H chain obtained by digesting the upper peptide side of two disulfide bonds cross-linking two H chains in the hinge regions of IgG with papain, and the full L chain.

Originally, a human antibody is an antibody naturally existing in the human body, but it also includes antibodies obtained from a human antibody phage library and a human antibody producing transgenic animal, prepared based on the recent genetical, cell-engineering and development-engineering advance.

The antibody existing in the human body can be obtained, for example, by isolating a human peripheral blood lymphocyte, immortalizing it by infection with EB virus or the like, followed by cloning, culturing a lymphocyte which produces the antibody, and purifying the antibody from the culture supernatant.

The human antibody phage library is a library in which an antibody fragment such as Fab, scFv or the like is expressed on the surface of a phage by inserting an antibody gene prepared from a human B cell into the phage gene. A phage which expresses an antibody fragment having a desired antigen binding activity can be recovered from the library as the measure of the binding activity to an antigen-immobilized substrate. The antibody fragment can be further genetically converted into a human antibody molecule comprising two complete H chains and two complete L chains.

A human antibody-producing transgenic animal is an animal in which a human antibody gene has been introduced into its cell. Specifically, a human antibody-producing transgenic animal can be produced by transfecting a human antibody gene into a mouse ES cell, transplanting the ES cell into an initial stage embryo of other mouse, and developing an animal. The human antibody may be produced and accumulated by obtaining a hybridoma producing a human antibody according to a hybridoma preparation method usually carried out in mammals other than human and then culturing the hybridoma to obtain the human antibody in a culture supernatant.

The Fab of the present invention can be obtained by treating an anti-KDR antibody specific for 1175-tyrosine phosphorylation with papain. Furthermore, the Fab can be produced by inserting DNA encoding an Fab fragment of the antibody into an expression vector for prokaryote or an expression vector for eukaryote, and introducing the vector into a prokaryote or eukaryote to express the Fab.

An Fab' is an antibody fragment having about 50,000 molecular weight and an antigen binding activity, which is obtained by cutting a disulfide bond of the hinge regions of the above F(ab')₂.

The Fab' of the present invention can be obtained by treating an anti-KDR antibody specific for 1175-tyrosine phosphorylation with a reducing agent, dithiotnreitol. Furthermore, the Fab' can be produced by inserting DNA encoding an Fab' fragment of the antibody into an expression vector for prokaryote or an expression vector for eukaryote, and introducing the vector into a prokaryote or eukaryote to express the Fab'.

An F(ab')₂ is a fragment having about 100,000 molecular weight and an antigen binding activity, which is constituted by two Fab regions bound at the hinge regions which are obtained by digesting the lower side of two disulfide bonds in the hinge regions of IgG with trypsin.

The F(ab')₂ of the present invention can be obtained by treating an anti-KDR antibody specific for 1175-tyrosine phosphorylation with trypsin. Furthermore, the F(ab')₂ can be produced by inserting DNA encoding an F(ab')₂ fragment of the antibody into an expression vector for prokaryote or an expression vector for eukaryote, and introducing the vector into a prokaryote or eukaryote to express the F(ab')₂.

An scFv is a VH-P-VL or VL-P-VH polypeptide in which one VH and one VL are linked using an appropriate peptide linker (hereinafter referred to as "P"). The VH and VL comprised in the scFv of the present invention may be any nati-KDR antibody specific for 1175-tyrosine phosphorylation of the present invention.

The scFv of the present invention can be produced by obtaining cDNAs encoding VH and VL from a hybridoma producing an anti-KDR antibody specific for 1175-tyrosine phosphorylation, constructing an scFv expression vector, inserting the cDNAs into the expression vector, and then introducing the expression vector into *Escherichia coli*, yeast or animal cell to express the scFv.

A diabody is an antibody fragment in which scFv's having the same or different antigen binding specificity forms a dimer. The diabody has a bivalent antigen binding activity to the same antigen or a bispecific antigen binding activity to different antigens.

The diabody of the present invention can be produced by obtaining cDNAs encoding VH and VL of an anti-KDR antibody specific for 1175-tyrosine phosphorylation of the present invention, constructing DNA encoding scFv having a polypeptide linker of 3 to 10 residues, inserting the DNA into an expression vector for prokaryote or an expression vector for eukaryote, and introducing the vector into a prokaryote or eukaryote to express the diabody.

A dsFv is an antibody in which polypeptides prepared by substituting one amino acid residue in each of VH and VL with a cysteine residue are linked via a disulfide bond. The amino acid residue substituted with a cysteine residue can be selected based on the three-dimensional structure estimation of the antibody in accordance with the method shown by Reiter *et al*. (*Protein Engineering,* 7, 697 (1994)). As the VH or VL comprised in the dsFv of the present invention, any anti-KDR antibody specific for 1175-tyrosine phosphorylation can be used.

The dsFv of the present invention can be produced by obtaining cDNAs encoding VH and VL from a hybridoma which produces an anti-KDR antibody specific for 1175-tyrosine phosphorylation, inserting the cDNAs into an expression vector for prokaryote or an expression vector for eukaryote, and introducing the vector into a prokaryote or cukaryote to express the dsFv.

A peptide comprising CDR includes at least one region of CDR of VH or VL. A peptide comprising plural CDRs can be produced by binding the CDRs directly or via an appropriate peptide linker.

The peptide comprising CDR of the present invention can be produced by constructing cDNA encoding CDR of VH or VL of an anti-KDR antibody specific for 1175-tyrosine phosphorylation, inserting the cDNA into an expression vector for prokaryote or an expression vector for eukaryote, and introducing the expression vector into a prokaryote or eukaryote to express the peptide. Furthermore, the peptide comprising CDR can also be produced by a chemical synthesis method such as an Fmoc method (fluorenylmethoxycarbonyl method), a tBoc method (t-butyloxycarbonyl method) or the like.

The anti-KDR antibody specific for 1175-tyrosine phosphorylation and the antibody fragment thereof according to the present invention include antibody derivatives in which a radioisotope, a protein or an agent is chemically or genetically conjugated to the antibody or the antibody fragment thereof.

The antibody derivatives of the present invention can be produced by chemically conjugating a radioisotope, a protein or a agent to the N-terminal side or C-terminal side of an H chain or an L chain of an anti-KDR antibody specific for 1175-tyrosine phosphorylation, to an appropriate substituent group or side chain of the antibody or the antibody fragment, or to a sugar chain in the antibody or the antibody fragment (*Antibody Engineering Handbook,* edited by Osamu Kanemitsu, published by Chijin Shokan (1994)).

Furthermore, it can be genetically produced by linking a DNA encoding an anti-KDR antibody specific for 1175-tyrosine phosphorylation or the antibody fragment thereof to other DNA encoding a protein to be conjugated, inserting the DNA into an expression vector, and introducing the expression vector into a host cell.

Examples of the isotope include ¹³¹I, ¹²⁵I and the like, and they can be conjugated to an antibody by, e.g., a chloramine T method.

As the agent, a low molecular weight agent is preferred. Examples include an anticancer agent such as an alkylating agent (e.g., nitrogen mustard, cyclophosphamide, *etc*.), a metabolic antagonist (e.g., 5-fluorouracil, methotrexate, *etc.*), an antibiotic (e.g., daunomycin, bleomycin, mitomycin C, daunorubicin, doxorubicin, *etc.*), a plant alkaloid (e.g., vincristine, vinblastine, vindesine, *etc.*), a hormone drug (e.g., tamoxifen, dexamethasone, *etc.*) and the like (*Clinical Oncology*, edited by Japanese Society of Clinical Oncology, published by Cancer and Chemotherapy (1996)); an anti-inflammatory agent such as a steroid agent (e.g., hydrocortisone, prednisone, *etc.*), a non-steroidal drug (e.g., aspirin, indometacin, *etc.*), an immunornodulator (e.g., aurothiomalate, penicillamine, *etc.*), an immunosuppressing agent (e.g., cyclophosphamide, azathioprine, *etc.),* an antihistaminic agent (e.g., chlorpheniramine maleate, clemastine, *etc.*) and the like (*Inflammation and Antiinflammatory Therapy*, Ishiyaku Shuppan (1982)); and the like. Examples of the method for conjugating daunomycin to an antibody include a method in which daunomycin and an amino group of an antibody are conjugated via glutaraldehyde, a method in which an amino group of daunomycin and a carboxyl group of an antibody are conjugated via a water-soluble carbodiimide and the like.

As the protein, cytokine which activates immunocompetent cells is preferred. Examples include human interleukin 2 (hIL-2), human granulocyte macrophage colony-stimulating factor (hGM-CSF), human macrophage colony-stimulating factor (hereinafter referred to as "hM-CSF"), human interleukin 12 (hIL-12) and the like. Also, in order to injure cancer cells directly, a toxin such as ricin, diphtheria toxin or the like can be used. For example, a fusion antibody with a protein can be produced by linking a cDNA encoding an antibody or the antibody fragment to other cDNA encoding the protein, constructing DNA encoding the fusion antibody, inserting the DNA into an expression vector for prokaryote or an expression vector for eukaryote, and then introducing it into a prokaryote or eukaryote to express the fusion antibody.

Examples of the compound which inhibits binding of a signal transduction molecule to 1175-tyrosine phosphorylated KDR/Flk-1 include an antisense KNA or DNA for 1175-tyrosine phosphorylated KDR/Flk-1, an antibody against the signal transduction molecule or the antibody fragment thereof, an antisense RNA or DNA for the signal transduction molecule, a compound obtained by a screening method or drug design method which will be described later and the like. Examples of the signal transduction molecule include PLC-γ.

Examples of the substance which inhibits phosphorylation of 1175-tyrosine of KDR/Flk-1 of the present invention include an antibody which inhibits phosphorylation of 1175-tyrosine of KDR/Flk-1 or the antibody fragment thereof, a kinase inhibitor having an activity of inhibiting phosphorylation of 1175-tyrosine of KDR/Flk-1, a compound obtained by a screening method or drug design method which will be described later and the like.

The substance which inhibits binding of a signal transduction molecule to 1175-tyrosine phosphorylated KDR/Flk-1, the substance which inhibits phosphorylation of 1175-tyrosine of KDR/Flk-1 and use of medicaments comprising the substance will be explained below.

### 1. Method for obtaining a substance which inhibits binding of a signal transduction molecule to 1175-tyrosine phosphorylated KDR/Flk-1 and a substance which specifically inhibits phosphorylation of 1175-tyrosine of KDR/Flk-1

### 1-1. Method for producing anti-KDR antibody specific for 1175-tyrosine phosphorylation

### (1) Preparation of peptide using as antigen

An anti-KDR antibody specific for 1175-tyrosine phosphorylation can be prepared by chemically synthesizing a peptide which comprises an amino acid sequence of continued 5 to 20 residues containing the 1175-tyrosine in the amino acid sequence of KDR/Flk-1 shown in SEQ ID NO:7, wherein the amino acid corresponding to the 1175-tyrosine is a phosphorylated tyrosine, and immunizing an animal with this peptide as the antigen. If necessary, a cysteine residue for binding to a carrier protein which will be described later may be added to the N-terminus or C-terminus of the peptide. Such an antigen peptide include a peptide having the amino acid sequence represented by SEQ ID NO:1 which has a sequence in which cysteine is added to the N-terminus of a sequence of the 1171st to 1180th of the amino acid sequence of KDR/Flk-1, wherein the 4th amino acid corresponding to the 1175-tyrosine residue is a phosphorylated tyrosine. A tyrosine-phosphorylated peptide can also be chemically synthesized by carrying out a solid phase synthesis using a peptide synthesizer based on the reported methods (Kitas EA *et al., Helv. Chim, Acta*, 74, 1314 (1991), Bannworth W and Kitas EA, *Helv. Chim. Acta*, 75, 707 (1992), Kitas EA *et al.*, *Tetrahedron Lett*., 30, 6229 (1989), Kitas EA *et al.*, *Tetrahedron Lett*., 29, 3591 (1988)).

### (2) Preparation of an anti-KDR polyclonal antibody specific for 1175-tyrosine phosphorylation

An antiserum comprising a polyclonal antibody against the peptide can be prepared by subcutaneously, intravenously or intraperitoneally administering the antigen together with an appropriate adjuvant to an animal such as mouse, rat, hamster, rabbit or the like for immunization several times at intervals of 10 days to 4 weeks based on the general method described in a reference such as (Harlow E and Lane D, *Antibodies: A Laboratory Manual*, Cold Spring Harbor Press (1988)) (hereinafter referred to as "*Antibodies: A Laboratory Manual*"). Since the peptide prepared in (1) alone has a weak activity as an antigen to induce antibodies, the peptide conjugated to a carrier protein such as keyhole limpet hemocyanine (KLH), bovine serum albumin, ovalbumin or tetanus toxin is used as the antigen and is administered at a dose of, e.g., 200 to 1,000 µg in the case of a rabbit, or 10 to 100 µg in the case of a mouse, per administration. The peptide can be bound to the carrier protein by using m-maleimidobenzoyl-N-hydroxysuccinimide (MBS) or glutaraldehyde. Examples of the adjuvant include complete Freund's adjuvant and aluminum hydroxide gel with pertussis vaccine. Three to ten days after each administration, a blood sample is taken from the venous plexus of the fundus of the eye or the tail vein of the immunized animal, its reactivity with the phosphorylated peptide used as the antigen is confirmed by enzyme immunoassay, and a serum sample collected from an animal whose serum showed a sufficient antibody titer is used as the antiserum. The enzyme immunoassay is a method in which the phosphorylated peptide used as the antigen is coated on a plate, allowed to react with a serum sample as a first antibody and further allowed to react with an anti-immunoglobulin antibody (an antibody against immunoglobulin of the animal used in immunization) as a second antibody labeled with, e.g., biotin, an enzyme, a chemiluminescent substance or an radioisotope, and then an antibody which recognizes the antigen peptide and binds thereto is detected and determined by carrying out a reaction in response to the labeled substance (*Enzyme Immunoassay (ELISA Method)*, published by Igaku Shoin (1976)).

A polyclonal antibody which binds to the antigen peptide can easily be purified by carrying out affinity chromatography for the antiserum using an affinity column prepared by immobilizing the peptide for antigen prepared in (1) on, e.g., Sepharose 4B (manufactured by Amersham Pharmacia Biotech) activated with N-hydroxysuccinimide (NHS), a column already packed with the resin (HiTrap NSH-activated column, manufactured by Amersham Pharmacia Biotech) or the like. Immobilization of the peptide and affinity chromatography can be carried out in accordance with the manufacture's instructions.

An antibody which can bind to not only the peptide used as the antigen and 1175-tyrosine phosphorylated KDR/Flk-1 to but also KDR/Flk-1 in which the 1175-tyrosine is not phosphorylated are included in the thus purified polyclonal antibody since the antibody recognizes a moiety other than the phosphorylated tyrosine in the sequence of the peptide used for the antigen as an epitope. Thus, the anti-KDR antibody specific for 1175-tyrosine phosphorylation can be purified by chemically synthesizing a peptide in which the phosphorylated tyrosine in the same amino acid sequence of the peptide for antigen prepared in (1) is changed to (not phosphorylated) tyrosine, passing the purified polyclonal antibody through an affinity column to which this peptide has been immobilized in the same manner, and thereby removing antibodies which also recognize KDR/Flk-1 in which the 1175-tyrosine is not phosphorylated (such antibodies also bind to an unphosphorylated tyrosine peptide on the affinity column) by binding them to the column.

### (3) Preparation of a monoclonal antibody

A 3- to 20-weeks-old mouse or rat is immunized in the same manner as (2), and antibody-producing cells are collected from spleen cells, lymph nodes and peripheral blood of an animal in which its serum shows a sufficient antibody titer.

As the melanoma cells, melanoma cells which can propagate *in vitro*, such as an 8-azaguanine resistant mouse (BALB/c derived) melanoma cell line P3-X63Ag8-U1 (P3-U1) (Kohler G *et al., European J. Immunol*., 6, 511 (1976)), SP2/0-Ag14 (SP-2) (Shulmanet M *et al*., *Nature,* 276, 269 (1978)), P3-X63-Ag8653 (653) (Keamey JF *et al., J. Immunology,* 123, 1548 (1979)), P3-X63-Ag8 (X63) (*Nature*, 256, 495 (1975)) and the like as established cells obtained from mice, can be used. Culturing and subculturing of these cell lines are carried out in accordance with known methods (*Antibodies: A Laboratory Manual)* to thereby secure a cell number of 2×10⁷ cells or more on the day of cell fusion.

Antibody-producing cells and myeloma cells are washed with a minimal essential medium (MEM) or PBS (phosphate buffered saline), a cell aggregating medium such as polyethylene glycol-1000 or the like is added thereto to carry out cell fusion. The fused cells are suspended in HAT medium {normal medium (a medium prepared by adding glutamine (1.5 mmol/l), 2-mercaptoethanol (5×10⁻⁵ mol/l), gentamicin (10 µg/ml) and fetal calf serum (FCS) (10%) to RPMT-1640 medium further supplemented with hypoxanthine (10⁻⁴ mol/l), thymidine (1.5×10⁻⁵ mol/l) and aminopterin (4×10⁻⁷ mol/l)} and cultured by dispensing the suspension into a 96-well plate.

After culturing, a portion of the culture supernatant in each well is taken out and measured by enzyme immunoassay to select cells which do not react with an unphosphorylated peptide having the same sequence of the antigen peptide of (2) but react specifically with the phosphorylated peptide used as the antigen. By repeating cloning twice by limiting dilution (using HT medium (a medium prepared by removing aminopterin from HAT medium) in the first cloning and the normal medium in the second cloning), a hybridoma showing a stably high antibody titer are selected from cells in the selected wells as a hybridoma cell line which produces an anti-KDR antibody specific for 1175-tyrosine phosphorylation.

The thus obtained hybridoma cells which produces an anti-KDR antibody specific for 1175-tyrosine phosphorylation are injected at a dose of 2×10⁷ to 5×10⁶ cells/animal into the abdominal cavity of a 8- to 10-weeks-old mouse or nude mouse reared for 2 weeks after intraperitoneal administration of 0.5 ml of pristane (2,6,10,14-tetramethylpentadecane). The hybridoma causes ascites tumor in 10 to 21 days. The ascitic fluid is collected from the mouse or nude mouse, and an IgG or IgM fraction is recovered to as the purified monoclonal antibody by carrying out centrifugation, salting out with 40 to 50% saturated ammonium sulfate or caprylic acid precipitation or using a column such as DEAE-Sepharose column, protein A column or Cellulofine GSL2000 column (manufactured by Seikagaku Corporation).

The subclass of the purified monoclonal antibody can be determined using a kit such as a monoclonal antibody typing kit A protein amount can be calculated by the Lowry method or absorbance at a wavelength of 280 nm.

### 1-2. Method for screening a substance which inhibits binding of a signal transduction molecule to 1175-tyrosine phosphorylated KDR/Flk-1 and a substance which specifically inhibits phosphorylation of 1175-tyrosine of KDR/Flk-1

A substance which inhibits binding of a signal transduction molecule to 1175-tyrosine phosphorylated KDR/Flk-1 or a substance which inhibits phosphorylation of 1175-tyrosine can be screened using the antibody obtained in 1-1 and KDR/Flk-1-expressing cells. Specifically, a substance which inhibits binding of a signal transduction molecule to 1175-tyrosine phosphorylated KDR/Flk-1 or a substance which inhibits phosphorylation of 1175-tyrosine can be selected by allowing the antibody to contact with a KDR/Flk-1-expressing cell and/or a tested substance and qualitatively or quantitatively examining properties such as binding of the antibody to KDR/Flk-1 and induction or inhibition of phosphorylation.

The tested substance is not particularly limited, so long as it can be added to a culture of KDR/Flk-1-expressing cells. Examples include a low molecular compound, a high molecular compound, an organic compound, an inorganic compound, a protein, a gene, a virus, a cell and the like. The tested samples excluding a gene may be added directly to the culture medium.

Examples of the method for efficiently introducing a gene into the culturing system include a method in which it is added to the culture by carrying on a virus vector such as retrovirus, adenovirus, adeno-associated virus, herpes simplex virus, lentivirus or the like, and a method in which it is added to the culture by including in an artificial vehicle structure. Examples include reports on the gene analysis using recombinant virus vectors (*Proc. Natl. Acad*. *Sci. USA*, 92, 6733 (1995); *Nucleic Acids Res*., 18, 3587 (1990); *Nucleic Acids Res*., 23, 3816 (1995)).

The screening method of the present invention includes a screening method using immunoassay. The immunoassay includes any known immunoassays. Specific examples of the immunoassay include a competitive method and a sandwich method (*Immunology Illustrated*, 5th edition (Nankodo)), and a sandwich method is preferred.

The screening method using a sandwich method is specifically a method in which a first antibody is bound to a solid phase, allowed to react with a tested substance and a KDR/Flk-1-expressing cell cultured using a VEGF-containing medium, and then allowed to react with a labeled second antibody.

As the antibodies used in the sandwich method, any of a polyclonal antibody and a monoclonal antibody may be used, or the above antibody fragments such as Fab, Fab', F(ab)₂ and the like may be used. As a combination of the two antibodies used in the sandwich method, it may be a combination of monoclonal antibodies or antibody fragments which recognize a different epitope or a combination of a polyclonal antibody with a monoclonal antibody or an antibody fragment, but a monoclonal antibody having a specific binding activity is preferable for carrying out high sensitivity sandwich ELISA. Examples include a combination of an antibody which recognizes an extracellular region of KDR/Flk-1 or the antibody fragment thereof with the anti-KDR antibody specific for 1175-tyrosine phosphorylation produced in I or the antibody fragment thereof. The antibody bound to a solid phase and the labeled antibody may be any of the two antibodies.

The labeling method includes labeling with a label such as an radioisotope, an enzyme, a fluorescent substance, a luminescent substance and the like, and labeling with an enzyme is preferred.

The screening method is specifically described below.

The anti-KDR antibody specific for 1175-tyrosine phosphorylation is dispensed in a 96-well plate and adhered thereto by incubation overnight at 4°C. After washing, PBS containing 1% bovine serum albumin is added thereto and incubated at room temperature for 1 hour for blocking of nonspecific adhesion. After washing with PBS, a cell extract of KDR/Flk-1 -expressing cells cultured using a medium which does not contain VEGF is dispensed, and then VEGF and a tested substance are added thereto for carrying out the reaction by activating KDR/Flk-I. After washing, the amount of KDR/Flk-1 bound to each well is measured by immunoassay using an antibody which recognizes an epitope different from that of the anti-KDR antibody specific for 1175-tyrosine phosphorylation, such as an anti-KDR/Flk-1 antibody which recognizes the extracellular region of KDR/Flk-1. By measuring and comparing the bound amount of KDR/Flk-1 when the substance is not added and the bound amount when the tested substance is added, a substance which inhibits phosphorylation of 1175-tyrosine or a substance which inhibits binding of 1175-tyrosine phosphate to an adapter molecule such as PLC-γ can be screened.

### 1-3. Method for obtaining a substance which inhibits binding of a signal transduction molecule to 1175-tyrosine phosphorylated KDR/Flk-1 and a substance which specifically inhibits phosphorylation of 1175-tyrosine of KDR/Flk-1 by drug design

A substance which inhibits binding of a signal transduction molecule to 1175-tyrosine phosphorylated KDR/Flk-1 of the present invention can be obtained by estimating a binding region of the intracellular region of KDR/Flk-1 and the signal transduction molecule through a computer simulation for structures of the intracellular region of 1175-tyrosine phosphorylated KDR/Flk-1 and the signal transduction molecule, based on data obtained by a structural analysis such as X-ray crystal analysis or NMR analysis, and by constructing compounds capable of inhibiting their intermolecular binding using a known data base or computer software.

Also, a substance which specifically inhibits phosphorylation of 1175-tyrosine of KDR/Flk-1 can be obtained by estimating a structure of the tyrosine kinase when 1175-tyrosine is phosphorylated, through a computer simulation based on data obtained by a structural analysis such as X-ray crystal analysis, NMR analysis or the like, and by selecting novel compounds from a known data base or constructing them using a computer software.

In this case, (1) when the three-dimensional structure of a structure-unknown protein is automatically constructed from the three-dimensional structure of a known analogous enzyme, for example, Insight II/Modeler (Accelrys) is used. (2) When the ligand binding site is estimated from the three-dimensional structure of the protein, for example, Cerius2/LigandFit (Accelrys) is used. (3) When the binding form of the ligand is estimated or when binding (inhibition) of an optional compound is estimated under such conditions that the three-dimensional structure of the protein is known and the ligand binding site is roughly known (as a region), for example, Insight II/Affinity (Accelrys), GOLD (CCDC), FlexX (Tripos) or DOCK (Prof. Kuntz) is used. (4) When a compound considered to bind (inhibit) to is produced from the ligand binding site, for example, Insight II/Ludi (Accelrys) is used.

Whether or not the thus obtained compound has an inhibition activity can be confirmed by using the assay shown in the screening method of 1-2.

### 2. Inhibition of KDR/Flk-1 signal transduction and inhibition of cell growth by an anti-KDR antibody specific for 1175-tyrosine phosphorylation

Inhibition of the KDR/Flk-1 signal transduction or inhibition of cell growth or angiogenesis by a substance which inhibits binding of a signal transduction molecule to 1175-tyrosine phosphorylated KDR/Flk-1 or a substance which specifically inhibits phosphorylation of 1175-tyrosine of KDR/Flk-1 can be confirmed by the following methods.

### (1) Inhibition of binding of PLC-γ and KDR/Flk-1

A KDR/Flk-1-expressing cell such as NIH3T3-KDR (Sawano A *et al., Cell Growth Differ*., 7, 213 (1996)) is stimulated with VEGF to activate KDR/Flk-1 and then a cell extract is prepared. PLC-γ or the SH2 domain of PLC-γ such as a fusion protein of GST (glutathione S transferase) and the N-terminal SH2 domain of PLC-γ (manufactured by Santa Cruz Biotechnology Inc.) is added and conjugated thereto. For the mixture to which the antibody has been added and the mixture to which the antibody has not been added, the PLC-γ or SH2 domain of PLC-γ is separated using a method such as immunoprecipitation, glutathione-immobilized beads or the like, and then immunoblotting is carried out using an anti-KDR/Flk-1 antibody to compare amounts of KDR/Flk-1 in the immunoprecipitation products. When the amount of KDR/Flk-1 in the immunoprecipitation product is reduced in the case of the addition of the antibody in comparison with the case of no addition, it is considered that the antibody inhibits binding of KDR/Flk-1 and PLC-γ *in vitro*.

Also, cells prepared by injecting or not injecting the antibody by microinjection into KDR/Flk-1-expressing vascular endothelial cells such as sinusoid endothelial cells or human umbilical cord vein endothelial cells are stimulated with VEGF and then cell extracts are prepared. Each of these cell extracts is subjected to immunoprecipitation using an anti-PLC-γ antibody in the same manner, and then immunoblotting of the immunoprecipitation product is carried out using an anti-KDR/Flk-1 antibody to compare amounts of KDR/Flk-1 in the immunoprecipitation products. When the amount of KDR/Flk-1 in the immunoprecipitation product is reduced in the case of the injection of the antibody in comparison with the case of no injection, it is considered that the antibody inhibits binding of KDR/Flk-1 and PLC-γ *in vivo*. The immunoprecipitation and immunoblotting can be carried out by the methods described in textbooks such as *Antibodies; A Laboratory Manual* and the like.

### (2) Inhibition of PLC-γ phosphorylation

Cell extracts prepared from endothelial cells into which the antibody has been injected or has not been injected in the same manner as (1) after stimulation with VEGF are subjected to immunoprecipitation of PLC-γ using an anti-PLC-γ antibody, and then immunoblotting of the immunoprecipitation product is carried out using an anti-phosphorylated tyrosine antibody to compare the amounts of phosphorylated PLC-γ in the immunoprecipitation products. When the amount of phosphorylated PLC-γ in the immunoprecipitation product is reduced in the case of the injection of the antibody in comparison with the case of no injection, it is considered that the antibody inhibits phosphorylation of PLC-γ, namely activation at PLC-γ, in *vivo*.

### (3) Inhibition of MAP kinase activation

Cell extracts prepared from endothelial cells into which the antibody has been injected or has not been injected in the same manner as (1) after stimulation with VEGF are subjected to immunoblotting using an antibody specific for phosphorylated MAP kinase to compare amounts of the phosphorylated MAP kinase. When the amount of the phosphorylated MAP kinase in the cells is reduced in the case of the injection of the antibody in comparison with the case of no injection, it is considered that the antibody inhibits phosphorylation of MAP kinase, namely activation of MAP kinase, *in vivo*.

### (4) Inhibition of cell growth

To vascular endothelial cells in which the antibody has been injected or has not been injected into the cells in the same manner as in (1), bromodeoxyuridine (BrdU) is added under stimulation with VEGF, and the newly synthesized DNA by the VEGF stimulation is labeled. The cells are fixed for tissue immunostaining, and the cells in which DNA is newly synthesized are detected by staining them with an anti-BrdU antibody. When the number of stained cells in the case of the injection of the antibody is reduced in comparison with the case of no injection, it can be said that the antibody is inhibiting synthesis of DNA, namely signal of cell growth, *in vivo*. Furthermore, the amount of the synthesized DNA is measured by labeling a newly synthesized DNA through culturing by adding [³H]-thymidine instead of BrdU, recovering the cells using a glass filter or the like, and then measuring the radioactivity of the cells. When the radioactivity in the case of the injection of the antibody is reduced in comparison with the case of no injection, it is considered that the antibody inhibits synthesis of DNA, namely signal of cell growth, *in vivo*.

### 3. Use of substance which inhibits binding of signal transduction molecule to 1175-tyrosine phosphorylated KDR/Flk-1 and substance which specifically inhibits phosphorylation of 1175-tyrosine of KDR/Flk-1

The substance which inhibits binding of a signal transduction molecule to 1175-tyrosine phosphorylated KDR/Flk-1 and the substance which specifically inhibits phosphorylation of 1175-tyrosine of KDR/Flk-1 of the present invention have an activity of inhibiting binding of KDR/Flk-1 to a signal transduction molecule and an activity of inhibiting cell growth or an activity of inhibiting angiogenesis. Substances having these properties can be used in the following applications.

### (1) Usage of a substance which inhibits binding of a signal transduction molecule to 1175-tyrosine phosphorylated KDR/Flk-1 and a substance which specifically inhibits phosphorylation of 1175-tyrosine of KDR/Flk-1

According to 2, the anti-KDR antibody specific for 1175-tyrosine phosphorylation of the present invention can be used for inhibiting binding of KDR/Flk-1 to a signal transduction molecule and for inhibiting cell growth or angiogenesis.

It can be used in diagnosing or treating diseases related to cell growth, such as growth of solid tumor, or diseases related to angiogenesis, such as metastasis formation, diabetic retinopathy, retinopathy of prematurity and the like.

Also, since KDR-Flk-1 is important for the signal transduction of cell growth in cells such as vascular endothelial cells and the like, the anti-KDR antibody specific for 1175-tyrosine phosphorylation can be used for the inhibition of growth of endothelium via KDR/Flk-1. Thus, when signal transduction of the human VEGF receptor KDR can be inhibited, it is useful in diagnosing or treating diseases whose morbid states progress by abnormal angiogenesis, such as solid tumor growth, metastasis formation, arthritis in rhewmatoid arthritis, diabetic retinopathy, retinopathy of prematurity, psoriasis and the like in human.

### (2) Agent comprising, as an active ingredient, a substance which inhibits binding of a signal transduction molecule to 1175-tyrosine phosphorylated KDR/Flk-1 or a substance which specifically inhibits phosphorylation of 1175-tyrosine of KDR/Flk-1

As such inhibitors, 1) a substance which inhibits binding of KDR/Flk-1 to VEGF, such as a neutralising antibody against an extracellular domain, soluble KDR/Flk-1 and the like and 2) a substance which inhibits intracellular kinase and thereby inhibits signal transduction thereafter such as a kinase inhibitor and the like have so far been known. Against these substances, a substance which inhibits binding of a signal transduction molecule to 1175-tyrosine phosphorylated KDR/Flk-1 or a Substance which specifically inhibits phosphorylation of 1175-tyrosine of KDR/Flk-1 having properties confirmed by the above 2 can be used as a VEGF-KDR/Flk-1 signal transduction inhibitor of a novel mechanism, that is, a cell growth inhibitor and an angiogenesis inhibitor.

A medicament comprising a substance which inhibits binding of a signal transduction molecule to 1175-tyrosine phosphorylated KDR/Flk-1 or a substance which specifically inhibits phosphorylation of 1175-tyrosine of KDR/Flk-1 can be administered alone as a therapeutic agent, but generally, it is preferable to provide it as a pharmaceutical preparation produced by an optional method well known in the technical field of pharmaceutics by mixing it with one or more pharmaceutically acceptable carriers.

It is preferable to use an administration route which is most effective in carrying out a treatment. Examples include oral administration and parenteral administration such as buccal, tracheal, rectal, subcutaneous, intramuscular or intravenous administration and the like. In an antibody-containing pharmaceutical formulation, intravenous administration is preferable.

Examples of dosage form include a spray, a capsule, a tablet, a granule, a syrup, an emulsion, a suppository, injection, an ointment, a tape and the like.

Examples of formulation suitable for oral administration include an emulsion, a syrup, a capsule, a tablet, a powder, a granule and the like.

A liquid preparation such as an emulsion and a syrup can be produced using, as additives, water; a saccharide such as sucrose, sorbitol, fructose *etc*.; glycol such as polyethylene glycol, propylene glycol *etc.;* oil such as sesame oil, olive oil, soybean oil *etc*.; an antiseptic such as p-hydroxybenzoic acid esters *etc.,* a flavor such as strawberry flavor, peppermint *etc.;* and the like.

A capsule, a tablet, a powder, a granule and the like can be produced using, as additives, a filler such as lactose, glucose, sucrose, mannitol *etc.;* a disintegrating agent such as starch, sodium alginate *etc*.; a lubricant such as magnesium stearate, talc *etc.;* a binder such as polyvinyl alcohol, hydroxypropylcellulose, gelatin *etc*.; a surfactant such as fatty acid ester *etc.;* a plasticizer such as glycerol *etc*.; and the like.

Examples of the pharmaceutical preparation suitable for parenteral administration include an injection, a suppository, a spray and the like.

An injection is prepared using a carrier such as a salt solution, a glucose solution, a mixture of both or the like.

A suppository is prepared using a carrier such as cacao butter, a hydrogenated fat, a carboxylic acid or the like.

Also, a spray is prepared using the compound or antibody as such or using a carrier which does not stimulate the buccal or airway mucous membrane of the patient and can facilitate absorption of the compound or antibody by dispersing it as fine particles.

Examples of the carrier include lactose, glycerine and the like. Depending on the properties of the compound or antibody and the used carrier, it is possible to produce a pharmaceutical preparation such as aerosol, dry powder or the like. In addition, the components exemplified as additives for oral preparations can also be added to the parenteral preparation.

Although the dose of the pharmaceutical composition varies depending on the conditions of each patient such as age and symptoms, it is administered at a dose of 0.1 to 20 mg/kg/day as each compound or antibody for mammals including human. When the compound and the antibody are simultaneously administered, they are administered once a day (single administration or every day administration) or intermittently 1 to 3 times a week or once during 2 to 3 weeks, by intravenous injection. When they are administered separately, they are administered at an optional interval once a day (single administration or every day administration) or intermittently 1 to 3 times a week or once during 2 to 3 weeks, by intravenous injection.

### (3) Early stage detection of angiogenesis

Tissue immunostaining of clinical tissue materials using the antibody of the present invention produced in 1-1 can be carried out using the fact that the antibody can selectively detect phosphorylated KDR/Flk-1, namely KDR/Flk-1 performing signal transduction. Since the stained area indicates that KDR/Flk-1 is activated, a region at an early stage of angiogenesis or where angiogencsis is going to occur can be detected by observing the stained area at a stage earlier than the detection of newly formed blood vessel. The tissue immunostaining can be carried out by the methods described in textbooks such as *Antibodies: A Laboratory Manual* and the like.

### (4) Method for determining whether nor not signal transduction in systems of VEGF-KDR/Flk-1 is inhibited

In order to determine whether or not a tested substance actually has an effect of inhibiting the signal transduction in systems of VEGF-KDR/Flk-1 in an animal, several weeks were required until the effect is determined because of the currently used method in which a tumor is transplanted into a non-human animal such as a mouse or the likc, and growth of the transplanted tumor or angiogenesis around the tumor is observed and compared in the cases of the administration and non-administration of the tested substance.

According to the present invention, whether or not a tested substance has an effect of inhibiting VEGF can be determined within a short period by the following method using the fact that the antibody of the present invention can selectively detect the signal transduction-performing KDR/Flk-1. As the tested substance, the substances exemplified in the screening method can be used.

Vascular endothelia of a mouse to which a tested substance has been administered and an unadministered mouse (control) are collected 1 to 2 days after administration of the tested substance and tissue immunostaining is carried out using the anti-KDR antibody specific for 1175-tyrosine phosphorylation. The amount stained by the antibody indicates the amount of phosphorylated KDR/Flk-1. Accordingly, when the stained amount of vascular endothelia of the mouse to which the tested substance has been administered is reduced in comparison with the stained amount of vascular endothelia of the unadministered mouse, it can be determined that the signal transduction of VEGF-KDR/Flk-1 system in the animal individual is inhibited.

The present invention is explained based on examples, but the present invention is not limited thereto.

### BRIEF DESCRIPTION OF THE DRAWINGS

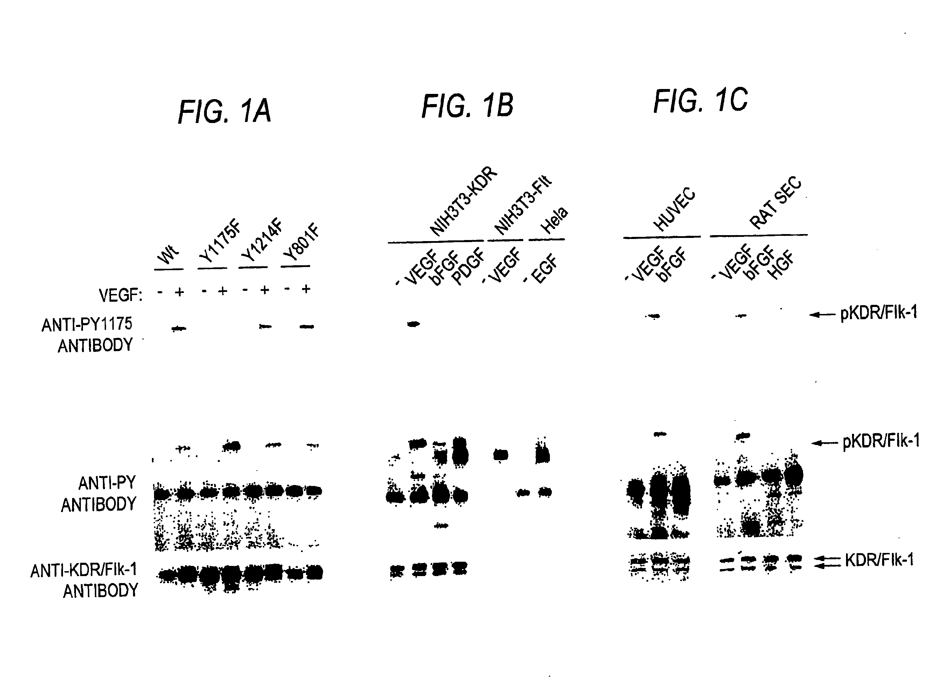

Fig. 1 is a drawing showing detection of 1175-tyrosine phosphorylated KDR/Flk-1 in various cells by immunoblotting using an antibody specific for phosphorylated 1175-tyrosine in KDR/Flk-1 (hereinafter referred to as "anti-PY1175 antibody"). Fig. 1A shows immunoblotting of cell extracts of a cell line MSS31 in which, from the left side, naturally occurring-type KDR/Flk-1 (Wt) and KDR/Flk-1 mutants Y1175F, yl214F and Y801F are expressed, "-" in each lane shows results using cell not stimulated with VEGF, and "+" shows results using cell stimulated with VEGF. Fig. 1B shows immunoblotting of cell extracts of, from the left side, NIH3T3-KDR, NIH3T3-Flt-1 and HeLa cells. "-" shows results using the cells not stimulated, and "VEGF", "bFGF", "PDGF" and "EGF" show results using the cells stimulated therewith, respectively. Fig. 1C shows immunoblotting of cell extracts of, from the left side, human umbilical cord vein endothelial cell (HUVEC) and rat sinusoid endothelial cell (ratSEC). "-" shows results using cells not stimulated, and "VEGF", "bFGF" and "HGF" show results using cells stimulated therewith, respectively. In each of Figs. 1A to 1C, the upper column shows results of immunoblotting using the anti-PY1175 antibody, the middle drawing shows results of immunoblotting using an anti-phosphorylated tyrosine antibody (anti-PY antibody), and the lower drawing shows results of immunoblotting using an anti-KDR/Flk-1 antibody. The arrows in the upper and middle columns show positions of the bands of auto-phosphorylated KDR/Flk-1 (pKDR/Flk-1), and the arrows in the lower column show positions of the bands of KDR/Flk-1 (including both auto-phosphorylated and not auto-phosphorylated).

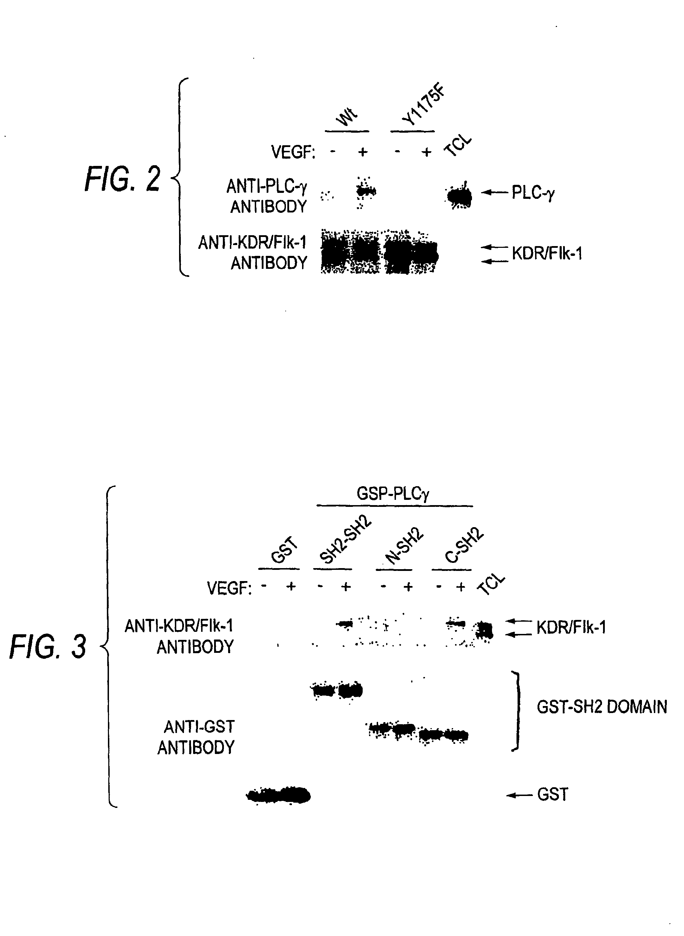

Fig. 2 is a drawing showing *in vivo* binding of KDR/Flk-l to PLC-γ in KDR/Flk-1-expressed MSS31 cells. The upper column shows results of immunoblotting using anti-PLC-γ antibody, and the lower column using anti-KDR/FIk-1 antibody, on anti-KDR/Flk-1 antibody immunoprecipitation products of respective cells wherein "Wt" is naturally occurring-type KDR/Flk-1, "Y1175F" is cells in which a Y1175F mutated KDR/Flk-1 is expressed, "-" is cells not stimulated with VEGF and "+" is cells stimulated with VEGF. The arrow in the upper column shows position of the band of PLC-γ, and the arrows in the lower column show positions of the bands of KDR/Flk-1. "TCL" shows results of immunoblotting of cell extracts of MSS31 cells (no immunoprecipitation) by an anti-PLC-γ antibody.

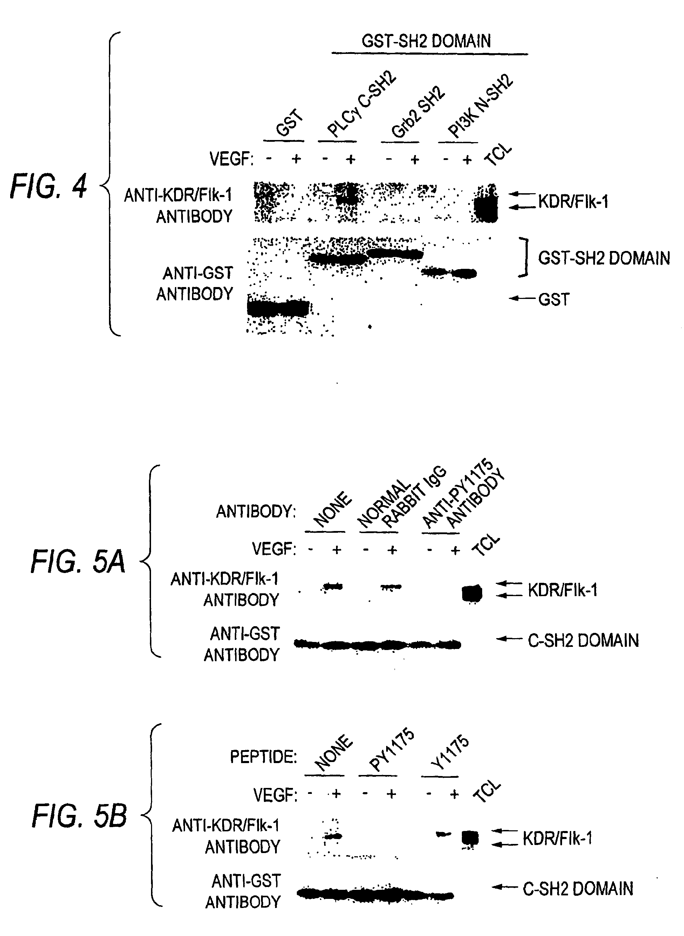

Fig. 3 is a drawing showing *in vitro* binding of the SH2 domain of PLC-γ to KDR/Flk-1. The upper column shows results of immunoblotting using an anti-KDR/Flk-1 antibody, and the lower column using an anti-GST antibody, on GST or a GST-SH2 domain fusion protein isolated using a glutathione affinity column after reacting GST or a fusion protein of GST and each SH2 domain of various proteins with cell extract of NIH3T3-KDR. "-" shows results using a cell extract of cells not stimulated with VEGF, and "+" shows results using a cell extract of cells stimulated with VEGF. Results using GST, GST-PLC-γ SH2-SH2, GST-PLC-γ N-SH2 and GST-PLC-γ C-SH2 are shown from the left side. The arrows in the upper column show positions of the band of KDR/Flk-1, and the arrow in the lower column shows position of the band of GST or a fusion protein of GST and each SH2 domain. TCL shows a result of immunoblotting of NIH3T3-KDR cell extract alone (no affinity column).

Fig. 4 is a drawing showing *in vitro* binding of the SH2 domain of PLC-γ to KDR/Flk-1, The upper column shows results of immunoblotting using an anti-KDR/Flk-1 antibody, and the lower column using an anti-GST antibody, on GST or a GST-SH2 domain fusion protein isolated using a glutathione affinity column after reacting GST or a fusion protein of GST and each SH2 domain of various proteins with a cell extract of NIH3T3-KDR. "-" shows results using a cell extract of cells not stimulated with VEGF, and "+" shows results using a cell extract of cells stimulated with VEGF. Results using GST, GST-PLC-γ C-SH2, GST-Grb2 SH2 and CST-PI3K N-SH2 are shown from the left side. The arrows in the upper column show positions of the band of KDR/Flk-1, and the arrow in the lower column shows position of the band of GST or a fusion protein of GST and each SH2 domain, TCL shows results of immunoblotting of a cell extract of NIH3T3-KDR alone (no affinity column).

Fig. 5 is a drawing showing inhibition of in vitro binding of the SH2 domain of PLC-γ to KDR/Flk-1 by an anti-PY1175 antibody and PY1175 peptide. The upper column shows results of immunoblotting using an anti-KDR/Flk-1 antibody, and the lower column using an anti-GST antibody, on GST-PLC-γ C-SH2 isolated after reacting GST-PLC-γ C-SH2 with a cell extract of NIH3T3-KDR and antibody/peptide. "-" shows results using a cell extract of cells not stimulated with VEGF, and "+" shows results using a cell extract of cells stimulated with VEGF. Fig. 5A shows results of, from the left side, no addition, rabbit IgG-added and anti-PY1175 antibody-added, and Fig. 5B shows results of, from the left side, no addition, PY1175 peptide-added and Y1175 peptide-added. The arrows in the upper column show positions of the band of KDR/Flk-1, and the arrow in the lower column shows position of the band of GST-PLC-γ C-SH2. TCL shows a result of immunoblotting of a cell extract of NIH3T3-KDR alone (no affinity column).

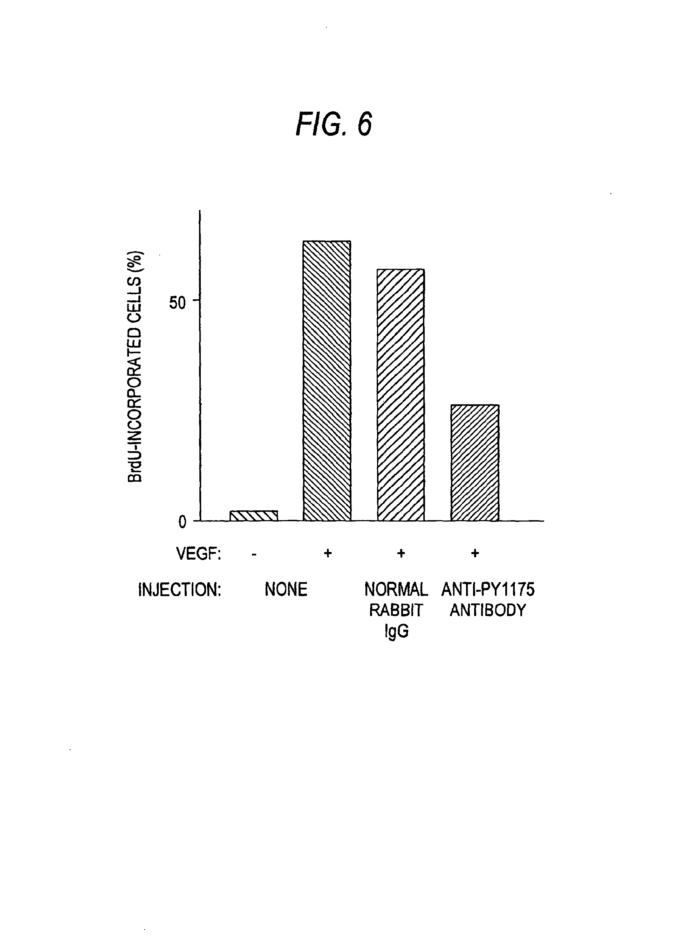

Fig. 6 is a drawing showing inhibition of a cell growth signal by VEGF in rat sinusoid endothelial cells into which an anti-PY1175 antibody has been injected. The graph shows ratios (%) of BrdU-incorporated cells of, from the left side, no antibody-injected (no VEGF stimulation), no antibody-injected (VEGF stimulation), rabbit IgG-injected (VEGF stimulation) and anti-PY1175 antibody-injected (VEGF stimulation).

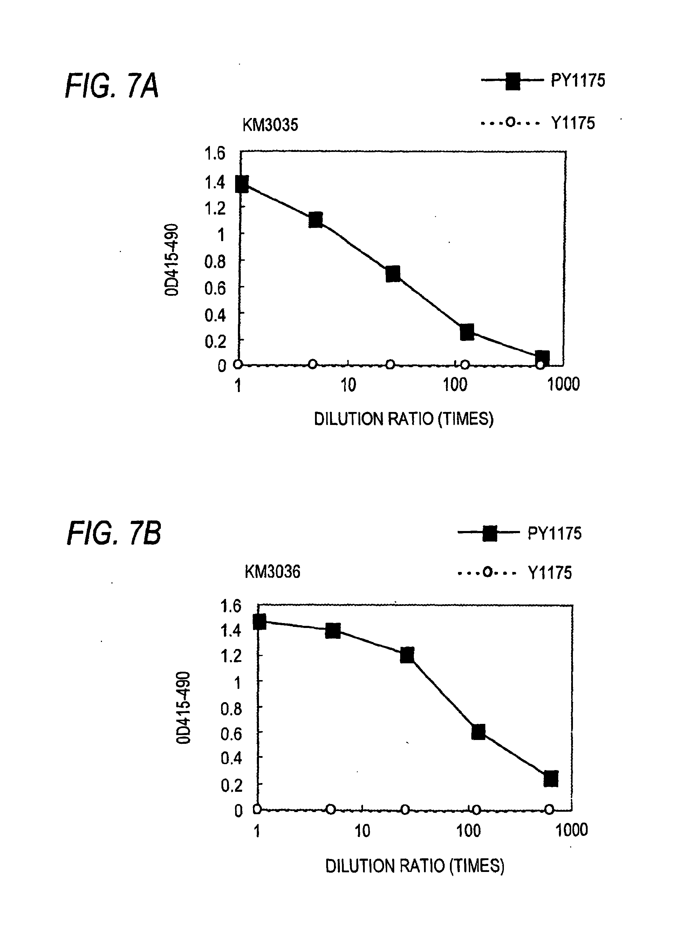

Fig. 7 is a drawing showing results in which a PY1175-THY- or Y1175-THY-adsorbed plate was allowed to react with culture supernatants containing, as a first antibody, monoclonal antibodies produced by hybridomas KM3035 or KM3036 or diluted solutions of the supernatant, and then allowed to react with a peroxidase-labeled rabbit anti-rat immunoglobulin as a second antibody.

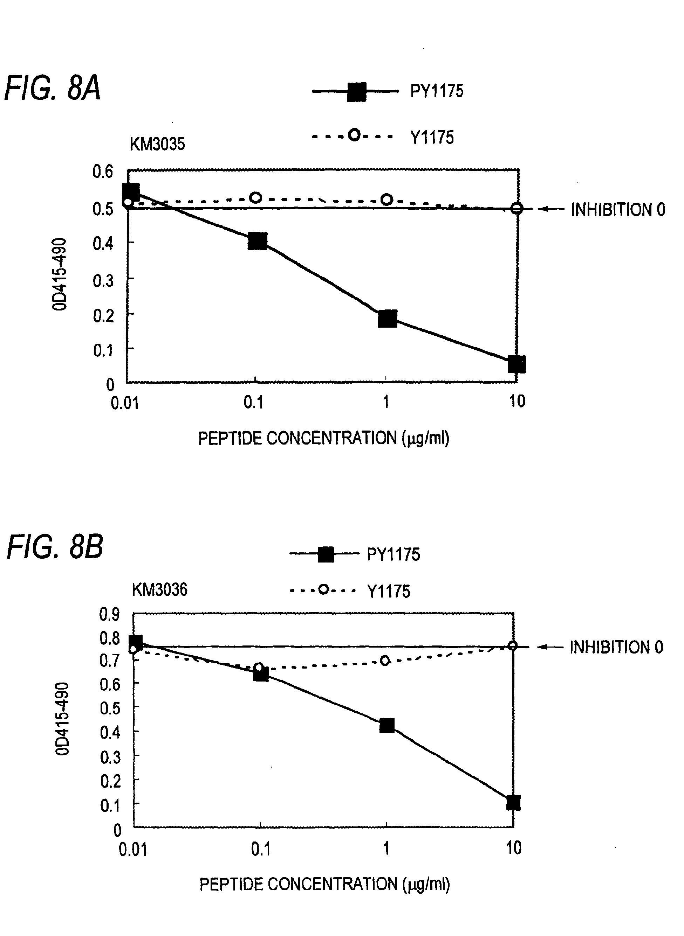

Fig. 8 is a drawing showing results in which each of stepwisely diluted PY1175 peptide and Y1175 peptide was dispensed into a plate prepared by immobilizing PY1175-THY thereon, a rabbit polyclonal antibody obtained by immunizing the animal with the synthetic peptide of positions 947 to 966 in KDR/Flk-1 obtained in Example 1 or a culture supernatant containing a monoclonal antibody produced by the hybridoma KM3035 was respectively dispensed into the resulting plate and allowed to react, a diluted peroxidase-labeled rabbit anti-rat immunoglobulin was added thereto and allowed to react, and then a color was developed using an ABTS (ammonium 2,2-azinobis(3-ethylbenzothiazole-6-sulfonate)) substrate solution.

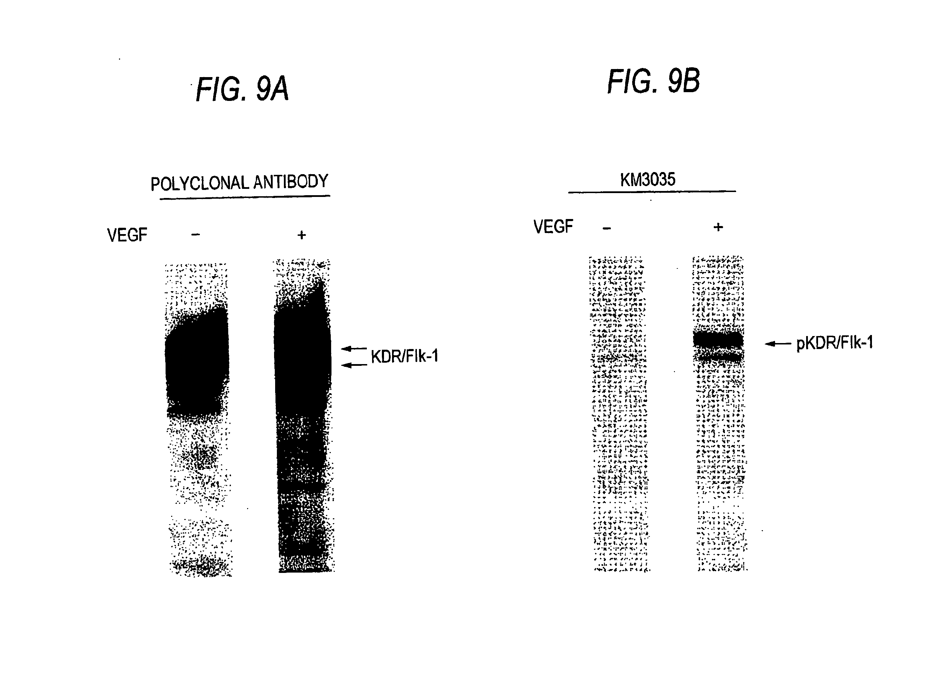

Fig. 9 is a drawing showing results in which cell extracts were prepared from VEGF-stimulated NIH3T3-KDR and VEGF-unstimulated control cells and immunoblotting was carried out using an anti-KDR/Flk-1 monoclonal antibody. Fig. 9A shows results using the anti-KDR/Flk-1 monoclonal antibody, and Fig. 9B shows results using an anti-PY1175 monoclonal antibody. "-" in each lane shows results using cells not stimulated with VEGF, and "+" shows results of cells stimulated with VEGF.

### BEST MODE FOR CARRYING OUT THE INVENTION

### Example 1

Antibody which can detect phosphorylation of 1175-tyrosine:

### (1) Preparation of an antibody which can specifically detect phosphorylation of 1175-tyrosine of KDR/Flk-1

A PY1175 peptide of the sequence shown in SEQ ID NO:1 (a sequence in which cysteine is added to the N-terminus of a sequence of positions 1171 to 1180 in the amino acid sequence of human KDR/Flk-1, wherein the tyrosine corresponding to position 1175 is phosphorylated) was chemically synthesized. An antiserum was obtained by immunizing a rabbit using an antigen prepared by conjugating the PY1175 peptide with KLH using cysteine. An antibody which binds to PY1175 was purified by passing the thus obtained antiserum through a PY1175 affinity column prepared by immobilizing the PY1175 peptide onto HiTrap NHS-activated column (manufactured by Amersham Pharmacia Biotech). Subsequently, a Y1175 peptide having the amino acid sequence represented by SEQ ID NO:2 (the same amino acid sequence of the PY1175, but the tyrosine is not phosphorylated) was chemically synthesized and immobilized onto a HiTrap NHS-activated column to prepare a Y1175 affinity column, and the antibody purified by the PY1175 affinity column was passed through this column to remove antibodies recognizing moieties other than the phosphorylated tyrosine as epitopes by binding them to the Y1175 affinity column to thereby purify only an antibody specific for the phosphorylated tyrosine of position 1175 in KDR/Flk-1 (nati-PY1175 antibody). Also, since the same sequence of positions 2 to 10 in PY1175 (Asp Gly Lys Asp Tyr Ile Val Leu Pro) is present in positions 1169 to 1179 of mouse KDR/Flk-1 (GenBank access No. X59397), it is considered that the thus prepared anti-PY1175 antibody can recognize not only human KDR/Flk-1 but also phosphorylated mouse KDR/Flk-1.

### (2) Specificity of anti-PY1175 antibody

A mouse endothelial cell line MSS31 cell (Yanai N *et al., Cell Struct. Func.,* 16, 87 (1991)) was cultured under the culturing conditions described in the above reference. This cell was infected with adenovirus vectors for the expression of naturally occurring-type KDR/Flk-1, each mutated KDR/Flk-1 of Y1175F in which the 1175-tyrosine was replaced by phenylalanine, Y1214F in which 1214-tyrosine was replaced by phenylalanine and Y801F in which 801-tyrosine was replaced by phenylalanine (cf., Reference Example 1) at 37°C for 1 hour. Each of these cells was cultured for 2 days after the infection, cultured for 12 hours by changing the medium to Dulbecco's modified Eagle's medium (DMEM) containing 0.1% FCS and then cultured at 37°C for 5 minutes by adding 10 ng/ml VEGF (a recombinant human VEGF purified from a culture supernatant of a VEGF expressing insect cell Sf-9 by heparin column chromatography in accordance with the description in a reference, Cohen T *et al., Growth Fact*., 7, 131 (1992)). From these cells and a control cell to which VEGF was not added, cell extracts were prepared using a cell lysis buffer (50 mmol/l HEPES (pH 7.4), 150 mmol/l NaCl, 10% glycerol, 1% Triton X-100, 5 mmol/l EDTA, 2% aprotinin, 1 mmol/l PMSF, 50 mmol/l NaF, 10 mmol/l sodium pyrophosphate, 2 mmol/l sodium vanadate), respectively. Each of the cell extracts was subjected to immunoblotting using the anti-PY1175 antibody. The immunoblotting hereinafter was carried out by a method in a reference (Takahashi T & Shibuya M, *Oncogene*, 14, 2079 (1997)). Also, immunoblotting was simultaneously carried out using an anti-KDR/Flk-1 antibody (a polyclonal antiserum produced by immunizing a rabbit using a chemically synthesized peptide of the sequence shown in SEQ ID NO:6 (corresponds to positions 947 to 966 in the amino acid sequence of human KDR/Flk-1) as the antigen) and an anti-phosphorylated tyrosine antibody (manufactured by ICN Biochemicals) to detect KDR/Flk-1 (independent of the presence or absence of phosphorylation) and a phosphorylated protein containing auto-phosphorylated KDR/Flk-1. From the results shown in Fig. 1A, the Y1175F which cannot be phosphorylated due to that position 1175 was phenylalanine was not detected in VEGF-added cells, whereas a band of each KDR/Flk-1 other than the Y1175F was detected with the anti-PY1175 antibody only in VEGF-added cells, and it was shown that 1175-tyrosine is phosphorylated VEGF-dependently inside the cells of endothelial cells. Also, since the band with the anti-PY1175 antibody was able to be detected only in the VEGF-added cells, it was confirmed that the anti-PY1175 antibody can specifically detect auto-phosphorylation of 1175-tyrosine in KDR/Flk-1.

Also, as the cells expressing other tyrosine kinase receptors such as bFGF receptor, PDGF receptor, VEGF receptor Flt-1, PDGF receptor and EGF receptor, NIH3T3-KDR (which expresses endogenous bFGF receptor and PDGF receptor in addition to the naturally occurring-type KDR/Flk-1; Sawano A *et al., Cell Growth Differ*., 7, 213 (1996)) as an NIH3T3 cell in which KDR/Flk-1 was constantly expressed, NIH3T3-Flt-1 (Sawano A *et al., Cell Growth Differ*., 7, 213 (1996)) as an NIH3T3 cell in which Flk-1 was constantly expressed and HeLa cell (which expresses endogenous EGF receptor, ATCC No. CCL-2) were cultured. Culturing of the NIH3T3-KDR and NIH3T3-Flt-1 was carried out using DMEM supplemented with 10% FCS, 2 mmol/l L-glutamine, 40 µg/ml kanamycin and 200 µg/ml G418, and the HeLa cell using DMEM supplemented with 10% FCS, 2 mmol/l L-glutamine and 40 µg/ml kanamycin. The serum concentration was reduced to 0.1%, and 6 to 7 hours after culturing, cell extracts of cells stimulated or not stimulated with respective ligands, VEGF, human bFGF (Oncogene Science Inc.), PDGF B/B (manufactured by Roche) and EGF (manufactured by Toyobo), were prepared and subjected to immunoblotting using the anti-PY1175 antibody, the anti-KDR/Flk-1 antibody and the anti-phosphorylated tyrosine antibody. The results are shown in Fig. 1B, in which ligand-dependent auto-phosphorylation of all tyrosine kinase receptors was detected with the anti-phosphorylated tyrosine antibody, whereas the band with the anti-PY1175 antibody was detected only when NIH3T3-KDR was stimulated with VEGF. Thus, it was confirmed that the anti-PY1175 antibody can specifically recognize the 1175-tyrosine phosphorylated KDR/Flk-1, which is different from the anti-phosphorylated tyrosine antibody which can detect all phosphorylated receptors.

### Example 2

### Detection of endogenous auto-phosphorylated KDR/Flk-1 by immunoblotting using an anti-PY1175 antibody:

A human umbilical cord vein endothelial cell (manufactured by Morinaga Biochemical Research Institute) expressing endogenous KDR/Flk-1 was cultured using a complete medium (manufactured by Nissui Pharmaceutical) or HuMedia-EG2 (manufactured by Kurabo Industries) supplemented with 5% FCS and various growth factors. Also, a rat sinusoid vascular endothelial cell expressing endogenous KDR/Flk-1 was isolated from rat kidney by the method described in a report (Yamane A *et al., Oncogene,* 9, 2683 (1994)) and cultured in HuMedia-EG2 containing 10 ng/ml VEGF. The medium was changed to DMEM containing 0.1% FCS, and after culturing these cells for 6 to 7 hours, they were stimulated by adding VEGF, bFGF or HGF (manufactured by R & D Systems Inc.). Cell extracts were prepared from the cells stimulated with, e.g., VEGF, and from un-stimulated control cells, and subjected to immunoblotting using the anti-PY1175 antibody, the anti-KDR/Flk-1 antibody and the anti-phosphorylated tyrosine antibody. The results are shown in Fig. 1C. A 230 kDa band of the 1175-tyrosine phosphorylated KDR/Flk-1 was detected by the anti-PY1175 antibody in both of the human umbilical cord vein endothelial cell and rat sinusoid vascular endothelial cell only when they were stimulated with VEGF. Thus, it was confirmed that 1175-tyrosine in endogenous KDR/Flk-1 in vascular endothelial cells is also auto-phosphorylated VEGF-dependently.

### Example 3

### Detection of auto-phosphorylated KDR/Flk-1 by tissue immunostaining using an anti-PY1175 antibody:

Human umbilical cord vein endothelial cells were cultured, the medium was changed to DMEM containing 0.1% FCS, and after culturing the cells for 6 to 7 hours, the cells were stimulated with 10 ng/ml VEGF or 50 ng/ml bFGF. The unstimulated control cells, cells 5 minutes after the VEGF stimulation, cells 30 minutes after the VEGF stimulation and cells 5 minutes after the bFGF stimulation were fixed respectively by treating them with acetone/methanol for 2 minutes. After blocking the fixed cells for 30 minutes with PBS containing 1% goat serum, tissue immunostaining was carried out using the anti-PY1175 antibody. The detection was carried out by fluorescence microscopic observation 30 minutes after treatment with an FITC-labeled anti-rabbit IgG antibody (manufactured by Cappel) diluted to 1/100. As a result, phosphorylation of the 1175-tyrosine of KDR/Flk-1 was detected in the cell membrane and cytoplasm of only cells 5 minutes after the VEGF stimulation, but phosphorylation of the 1175-tyrosine was no longer detected in the cells 30 minutes after the VEGF stimulation. Thus, it was found that the 1175-tyrosine was immediately phosphorylated by the VEGF stimulation, but this phosphorylation is transient. Since the unstimulated cells and bFGF-stimulated cells were not stained, this phosphorylation of the 1175-tyrosine was specific for the VEGF stimulation. Also, the staining was inhibited when 1 µg/ml of the PY1175 peptide was allowed to coexist at the time of the tissue immunostaining of cells 5 minutes after the VEGF stimulation. Accordingly, the anti-PY1175 antibody was useful for the detection of the 1175-tyrosine phosphorylation of KDR/Flk-1 by not only immunoblotting but also tissue immunostaining.

### Example 4

### Inhibition of binding of PLC-γ to KDR/Flk-1 by anti-PY1175 antibody:

### (1) Intracellular binding of KDR/Flk-1 and PLC-γ

In the same manner as in Example 1(2), naturally occurring-type KDR/Flk-1 or Y1175F was expressed in MSS31 cell using an adenovirus vector and stimulated with 10 ng/ml VEGF. Cell extracts were prepared from this cell and a control cell which was not stimulated with VEGF, and immunoprecipitation was carried out by the method described in a report (Takahashi T & Shibuya M, *Oncogene,* 14, 2079 (1997)) using the anti-KDR/Flk-1 antibody. The immunoprecipitation products were subjected to immunoblotting using an anti-PLC-γ antibody (manufactured by Upstate Biotechnology Inc.). The results are shown in Fig. 2. Since PLC-γ was detected in the immunoprecipitation product of the naturally occurring-type KDR/Flk-1-expressing cell only when stimulated with VEGF, it was confirmed that PLC-γ binds to KDR/Flk-1 by the VEGF stimulation. On the other hand, PLC-γ was not detected in the Y1175F-expressing cell even by the VEGF stimulation, so that it was found that PLC-γ does not bind to Y1175F. Thus, it was found that phosphorylation of the 1175-tyrosine is essential for the binding of PLC-γ.

### (2) In vitro binding of KDR/Flk-1 and PLC-γ SH2 domain

In the same manner as in Example 1(2), cell extracts were prepared from NIH3T3-KDR stimulated with 10 ng/ml VEGF and a control cell which was not stimulated with VEGF. Onto glutathione-Sepharose 4B beads (manufactured by Amersham Pharmacia Biotech), 1.5 µg of GST, a fusion protein of GST and SH2 domain (2 domains) of PLC-γ (GST-PLC-γ SH2-SH2), a fusion protein of GST and SH2 domain of the N-terminal side of PLC-γ (GST-PLC-γ N-SH2) and a fusion protein of GST and SH2 domain of the C-terminal side of PLC-γ (GST-PLC-γ C-SH2) (all manufactured by Santa Cruz Biotechnology Inc.) was immobilized respectively, and the cell extracts were added thereto, followed by incubation at 4°C for 2 hours. The beads were washed three times with a cooled cell extraction buffer and heated in an SDS sample buffer to dissolve the protein bound to the beads, and then SDS-PAGE was carried out. The KDR/Flk-1 bound to each of the added proteins was detected by immunoblotting using the anti-KDR/Flk-1 antibody. The results are shown in Fig. 3. The KDR/Flk-1 was detected in the VEGF-stimulated cell with GST-PLC-γ SH2-SH2 and GST-PLC-γ C-SH2, but KDR/Flk-1 was not detected with GST-PLC-γ N-SH2. Thus, it was found that among two SH2 domains of PLC-γ, the C-terminal side SH2 domain binds to the auto-phosphorylated KDR/Flk-1.

When the same test was carried out using GST, GST-PLC-γ C-SH2, a fusion protein of GST and SH2 domain of Grb2 (GST-Grb2 SH2) and a fusion protein of GST and SH2 domain of the N-terminal side of PI3 kinase (GST-PI3K N-SH2) (all manufactured by Santa Cruz Biotechnology Inc.), as shown in Fig. 4, KDR/Flk-1 was detected in the cell stimulated with VEGF only in the case of GST-PLC-γ C-SH2, but KDR/Flk-1 was not detected in the case of GST-Grb2 SH2 and GST-PI3K N-SH2. Thus, it was confirmed that the auto-phosphorylated KDR/Flk-1 binds to the SH2 domain of PLC-γ, but does not bind to the SH2 domains of Grb2 and PI3 kinase.

### (3) Inhibition of binding of KDR/Flk-1 and PLC-γ by an anti-PY1175 antibody

The same test of (2) was carried out by allowing the anti-PY1175 antibody and the PY1175 peptide to coexist at the time of the reaction of GST-PLC-γ C-SH2-immobilized beads with the cell extract. As a result, KDR/Flk-1 was not detected even in the VEGF-stimulated cell, and binding of the auto-phosphorylated KDR/Flk-1 and PLC-γ was inhibited by the anti-PY1175 antibody and the PY1175 peptide. On the other hand, the inhibition was not found when a control rabbit IgG or the Y1175 peptide was coexisted (Fig. 5A, Fig. 5B). Thus, it was strongly suggested that the C-terminal side SH2 domain of PLC-γ directly binds to the auto-phosphorylated 1175-tyrosine of KDR/Flk-1.

### Example 5

### Inhibition of cell growth signal by an anti-PY1175 antibody

Primary-cultured rat sinusoid endothelial cells were put on a slide glass coated with collagen, cultured for 2 days using a medium prepared by adding a growth additive for HuMedia-EG2 preparation (manufactured by Kurabo Industries) and VEGF to DMEM from which thymidine was excluded and then cultured for 6 to 7 hours using the same medium to which VEGF was not added. The cells were stimulated by adding 50 ng/ml VEGF and at the same time 100 µmol/l 5-bromodeoxyuridine (BrdU, manufactured by Sigma Aldrich) was added thereto to incorporate it into newly synthesized DNA by culturing the cells for 20 hours. Cells without adding VEGF as a control were also treated in the same manner. Next, respective cells were fixed with PBS containing 3.7% formaldehyde and treated with methanol for 10 minutes and 2 mol/l hydrochloric acid for 10 minutes to increase transparency of the cells. By carrying out tissue immunostaining by allowing the cells to react for 60 minutes with an anti-BrdU antibody (manufactured by Takara Shuzo) diluted to 1/200 with PBS containing 1% goat serum, BrdU-incorporated cells were detected. As shown the results in Fig. 6, incorporation of BrdU was hardly observed in the cells which were not stimulated with VEGF, whereas BrdU-incorporated cells were observed in large numbers when VEGF was added. It was confirmed that the cell growth signal in rat sinusoid endothelial cells is markedly depending on the VEGF stimulation.

Accordingly, rat sinusoid endothelial cells cultured on a slide glass in the same manner were cultured for 6 to 7 hours using the medium to which VEGF was not added and then, before the VEGF stimulation, the anti-PY1175 antibody or a control rabbit IgG was injected into the cytoplasm of each cell using automatic Micro-injector 5246 (manufactured by Eppendorf) and Micro-manipulator 5171 (manufactured by Eppendorf). One to three hours after the injection, 50 ng/ml VEGF and 100 µmol/l BrdU were added to the cells, followed by culturing for 20 hours to incorporate it into newly synthesized DNA in the same manner. In the same manner as above, the cells were fixed, treated with methanol and hydrochloric acid, allowed to react for 60 minutes with an anti-BrdU antibody (manufactured by Takara Shuzo) diluted to 1/200 and then allowed to react for 30 minutes with a mixture of 1/200 times-diluted FITC-labeled antimouse IgG (reacts with the anti-BrdU antibody, manufactured by Cappel) and 1/100 times-diluted RITC-labeled anti-rabbit IgG (anti-PY1175 antibody, which reacts with rabbit IgG, manufactured by Cappel), thereby carrying out tissue immunostaining and detecting the BrdU-incorporated cells and the cells into which the antibody (anti-PY1175 antibody or rabbit IgG) was injected. As a result shown in Fig. 6, BrdU was incorporated into half the number or more of the rabbit IgO-injected cells by the VEGF stimulation. Thus it was shown that the cell growth signal was transferred by the VEGF stimulation and DNA was synthesized. On the other hand, BrdU was incorporated into only about 1/4 of the cells into which the anti-PY1175 antibody was injected. Thus it was shown that the cell growth signal by the VEGF stimulation was inhibited.

### Example 6

### Production of a monoclonal antibody which can specifically detect phosphorylation of 1175-tyrosine of KDR/Flk-1:

The antibody specific for PY1175 obtained in Example 1 can be obtained by passing a polyclonal antibody against the phosphorylated tyrosine of position 1175 in KDR/Flk-1 through an Y1175 affinity column, but a large amount of the polyclonal antibody is required for obtaining an antibody which reacts only with PY1175 from the polyclonal antibody. Accordingly, a monoclonal specific for PY1175 was prepared.

### (1) Preparation of immunogen

The PY1175 peptide represented by the amino acid sequence shown in SEQ ID NO:1 was prepared by the following method for the purpose of increasing its immunogenicity.

First, KLH (manufactured by Calbiochem) was dissolved in PBS to give a concentration of 10 mg/ml, 1/10 volume of 25 mg/ml MBS (N-(m-maleimidobenzoyloxy)succinimide, manufactured by Nakalai Tesque) was added dropwise thereto, followed by stirring for reaction for 30 minutes. KLH-MBS (2.5 mg) obtained by removing free MBS using a gel filtration column such as Sephadex G-25 column or the like which had been equilibrated in advance with PBS was mixed with 1 mg of the PY1175 peptide dissolved in 0.1 M sodium phosphate buffer (pH 7.0), followed by stirring for reaction for 30 minutes. After the reaction, the mixture was dialyzed against PBS and used as the immunogen.

### (2) Immunization of an animal and preparation of antibody-producing cells

The KLH conjugate (50 µg) of the PY1175 compound prepared in Example 6(1) was administered to each of three 4-weeks-old female SD rats together with 2 mg of an aluminum hydroxide adjuvant (*Antibodies: A Laboratory Manual*, p. 99) and 1×10⁹ cells of pertussis vaccine (manufactured by Chiba Serum Institute). Two weeks after the administration, 50 µg of the KLH conjugate was administered once a week at a total of 4 times. A blood sample was collected from the heart of each rat, its serum antibody titer was examined by the following enzyme immunoassay, and the spleen was excised from a rat which showed a sufficient antibody titer 3 days after the final immunization.

The spleen was cut to pieces in MEM (minimum essential medium) (manufactured by Nissui Pharmaceutical), and cells were unbound using a pair of forceps and centrifuged (250×g, 5 minutes). To the thus obtained precipitation fraction, a Tris-ammonium chloride buffer (pH 7.6) was added thereto and treated for 1 to 2 minutes for eliminating erythrocytes. The thus obtained precipitation fraction (cell fraction) was washed three times with MEM medium and used in the cell fusion.

### (3) Enzyme immunoassay (binding ELISA)

The PY1175 and Y1175 obtained in Example 1(1) were conjugated with thyroglobulin (hereinafter referred to as "THY") and the conjugates (hereinafter referred to as "PY1175-THY" and "Y1175-THY", respectively) were used as the antigens for assay. The preparation method was described in Example 6(1), except that SMCC (4-(N-maleimidomethyl)cyclohexane-1-carboxylic acid N-hydroxysuccinimide ester; manufactured by Sigma) was used as the crosslinking agent instead of MBS. The thus prepared PY1175-THY or Y1175-THY was dispensed in 5 µg/ml (50 µl/well) into a 96-well EIA plate (manufactured by Greiner) and incubated overnight at 4°C to effect their adhesion. After washing the plate, 1% bovine serum albumin (BSA)/Dulbecco phosphate buffered saline (PBS) was dispensed in 100 µl/well and incubated at room temperature for 1 hour to thereby block the remaining active groups.

After the incubation, 1% BSA/PBS was discarded and an immunized rat antiserum or a hybridoma culture supernatant was dispensed as a first antibody in 50 µl/well portions into the plate and incubated for 2 hours. After washing the plate with 0.05% polyoxyethylene(20)sorbitan monolaurate (equivalent to Tween 20 (trademark of ICI): manufactured by Wako Pure Chemical Industries)/PBS (hereinafter referred to as "Tween-PBS"), peroxidase-labeled rabbit anti-rat immunoglobulin was dispensed as a second antibody in 50 µl/well and incubated for 1 hour. After washing the plate with Tween-PBS, color development was carried out by adding ABTS substrate solution (1 mmol/l ABTS dissolved in 0.1 mol/l citrate buffer (pH 4.2)) and the absorbance at OD415 nm was measured using a plate reader (Emax; manufactured by Molecular Devices).

### (4) Preparation of mouse myeloma cells

An 8-azaguanine-resistant mouse myeloma cell line P3X63Ag8-U1 (P3-U1; purchased from ATCC) was cultured using a normal medium (RPMI-1640 medium supplemented with 10% calf serum) to secure 2×10⁷ or more of the cells which were used as the parent cell line for cell fusion.

### (5) Preparation of hybridoma

The rat spleen cells obtained in Example 6(2) and the myeloma cells obtained Example 6(4) were mixed in a proportion of 10:1 and centrifuged (250×g, 5 minutes). Cells in the thus obtained precipitation fraction were thoroughly loosened, a mixed solution of 1 g of polyethylene glycol-1000 (PEG-1000), 1 ml of MEM and 0.35 ml of dimethyl sulfoxide was added to the cells in an amount of 0.5 ml per 10⁸ mouse spleen cells under stirring at 37°C, and then 1 ml of MEM medium was added several times at 1 to 2 minute intervals and the total volume was adjusted to 50 ml by adding MEM medium.

After centrifugation of the suspension (900 rpm, 5 minutes), cells in the thus obtained precipitation fraction are gently loosened and then suspended in 100 ml of HAT medium (a medium prepared by supplementing the 10% fetal calf serum with HAT Mcdia Supplement (manufactured by Invitrogen)) by gently repeated drawing up into and discharging from a measuring pipette. The suspension was dispensed in 200 µl/well into a 96 well culture plate and cultured at 37°C for 10 to 14 days in a 5% CO₂ incubator.

After culturing, the culture supernatants were examined by the enzyme immunoassay described in Example 6(3), and wells which reacted with the PY1175 peptide but did not react with the Y1175 peptide were selected. However, most of the cell culture supernatants reacted with both of PY1175 and Y1175. Accordingly, this procedure was repeated to select wells which reacted with the PY1175 peptide but did not react with the Y1175 peptide and cloning from the cells contained therein was repeated twice by limited dilution to thereby establish hybridomas KM3035 and KM3036 which produces an anti-PY1175 monoclonal antibody. The hybridoma KM3035 has been deposited on September 13, 2001 in International Patent Organism Depositary, National Institute of Advanced Industrial Science and Technology (AIST Tsukuba Central 6, 1-1, Wgashi 1-Chome Tsukuba-shi, Ibaraki-ken 305-8566 Japan) as PERM BP-7729.

### (6) Purification of a monoclonal antibody

Each of the hybridomas obtained in Example 6(5) was intraperitoneally injected into pristane-treated 8-weeks-old female nude mice (BALB/c) at a dose of 5 to 20×10⁶ cells/animal. The hybridoma causes ascites tumor in 10 to 21 days, and ascitic fluid was collected from a mouse filled with ascitic fluid caused (1 to 8 ml/animal).

The ascitic fluid was centrifuged (1,200×g, 5 minutes) to remove solid components.

Purified IgG monoclonal antibodies were obtained by purifying them by the caprylic acid precipitation method (*Antibodies: A Laboratory Manual*). Subclass of the monoclonal antibodies were determined by the ELISA using a subclass typing kit. Subclass of both of the monoclonal antibody KM3035 and monoclonal antibody KM3036 was IgG2a.

### Example 7

### Examination on the reactivity of monoclonal antibodies

### (1) Reactivity with an antigen compound in an antigen solid phase model (binding ELISA)

Reactivity of the monoclonal antibodies obtained in Example 6 with antigen peptides was examined in accordance with the method shown in Example 6(3). A plate to which PY1175-THY or Y1175-THY was adsorbed was allowed to react with each of culture supernatants containing the monoclonal antibodies produced by hybridomas KM3035 and KM3036 obtained in Example 6(5) or with each of dilutions prepared by diluting the culture supernatants 5¹, 5², 5³ and 5⁴ times, as a first antibody, and then allowed to react with peroxidase-labeled rabbit anti-rat immunoglobulin as a second antibody. The results are shown in Fig. 7. Both of the monoclonal antibodies produced by hybridomas KM3035 and KM3036 reacted only with the PY1175 peptide and did not react with the Y1175 peptide.

### (2) Reactivity with an antigen compound in an antigen liquid phase model (inhibition ELISA)

A plate to which PY1175-THY was immobilized in accordance with the method shown in Example 6(3) was prepared, each of the PY1175 peptide and the Y1175 peptide diluted from 20 µg/ml by 5-fold serial dilution was dispensed in 50 µl/well, and then each of culture supernatants containing the monoclonal antibodies produced by hybridomas KM3035 and KM3036 was diluted (dilution rate; KM3035: ×120, KM3036: ×1,000) and dispensed in 50 µl/well, followed by incubation for reaction at room temperature for 2 hours. After washing the wells with Tween-PBS, diluted peroxidase-labeled rabbit anti-rat immunoglobulin (manufactured by Dako) was dispensed in 50 µl/well and allowed to react at room temperature for 1 hour, and then, after washing with Tween-PBS, color development was carried out by adding ABTS substrate solution and the absorbance at OD415 nm was measured using a plate reader (Emax; manufactured by Wako Pure Chemical Industries).

As shown in Fig. 8, both of the monoclonal antibodies produced by hybridomas KM3035 and KM3036 reacted only with the PY1175 peptide in the liquid phase model.

### Example 8

### Detection of auto-phosphorylated KDR/Flk-1 by immunoblotting using monoclonal antibody KM3035

In the same manner as in Example 1(2), cell extracts were prepared from NIH3T3-KDR stimulated with 10 ng/ml VEGF and a VEGF-unstimulated control cell, and immunoblotting was carried out using an anti-KDR/Flk-1 monoclonal antibody.

As shown in Fig. 9, a 230 kDa band of KDR/Flk-1 was detected independent of the VEGF stimulation or unstimulation by the rabbit polyclonal antibody obtained by immunizing a rabbit with a peptide having an amino acid sequence of positions 947 to 966 in KDR/Flk-1.

On the other hand, in the monoclonal antibody KM3035, only when it was stimulated with VEGF, the 230 kDa band of phosphorylated KDR/Flk-1 was detected. Thus, it was confirmed that KM3035 can detect auto-phosphorylation of 1175-tyrosine of KDR/Flk-1.

### Reference Example 1

### Production of mutated KDR/Flk-1 expression adenovirus vector:

Adenovirus vectors for expressing mutated KDR/Flk-1 of Y1175F (1175-tyrosine was replaced by phenylalanine), Y1214F (1214-tyrosine was replaced by phenylalanine) and Y801F (801-tyrosine was replaced by phenylalanine) were prepared in the following manner.

A DNA fragment encoding Y1175F was prepared by carrying out PCR using a primer having the nucleotide sequence represented by SEQ ID NO:3 and using a naturally occurring-type KDR/Flk-1 cDNA (Sawano A *et al., Cell Growth Differ*., 7, 213 (1996)) as the template, and inserting the thus obtained transgenic fragment between the *Apa*I/*Pst*I site of KDR/Flk-1 cDNA. A DNA fragment encoding Y1214F was prepared by carrying out PCR in the same manner using a primer having the nucleotide sequence represented by SEQ ID NO:4, and inserting the thus obtained transgenic fragment between the *Apa*I/*Pst*I site of KDR/Flk-1 cDNA. Also, a DNA fragment coding for Y801F was prepared by carrying out PCR in the same manner using a primer having the nucleotide sequence represented by SEQ ID NO:5, and inserting the thus obtained transgenic fragment between the *Sph*I/*Bam*HI site of KDR/Flk-1 cDNA. It was confirmed that respective mutation was introduced into each of these DNA fragments encoding mutation type KDR/Flk-1 by determining their nucleotide sequences. Respective KDR/Flk-1 expression adenovirus vectors (recombinant adenovirus) were prepared using these mutated KDR/Flk-1-encoding DNA fragments with an adenovirus expression vector kit (manufactured by Takara Shuzo).

### INDUSTRIAL APPLICABILITY

The present invention provides a substance which blocks intracellular KDR/Flk-1 signal transduction and inhibits cell growth by VEGF. Furthermore, the present invention provides a method for inhibiting KDR/Flk-1 signal transduction, a method for inhibiting cell growth, a method for inhibiting angiogenesis, a method for screening a cell growth inhibitor, a method for screening an angiogencsis inhibitor, a method for screening a substance which inhibits KDR/Flk-1 signal transduction, a method for determining whether or not a tested substance can inhibit KDR/Flk-1 signal transduction, a method for detecting angiogenesis in tissue, and a method for screening a substance which inhibits 1175-tyrosine phosphorylated KDR/Flk-1, which comprise using the substance.

### FREE TEXT OF SEQUENCE

SEQ ID NO:1 - Explanation of artificial sequence: Synthetic amino acids
SEQ ID NO:2 - Explanation of artificial sequence: Synthetic amino acids
SEQ ID NO:3 - Explanation of artificial sequence: Synthetic DNA
SEQ ID NO:4 - Explanation of artificial sequence: Synthetic DNA
SEQ ID NO:5 - Explanation of artificial sequence: Synthetic DNA

## Claims

1. A method for inhibiting KDR/Flk-1 signal transduction, which comprises using a substance which inhibits binding of a signal transduction molecule to 1175-tyrosine phosphorylated KDR/Flk-1.

2. A method for inhibiting cell growth, which comprises using a substance which inhibits binding of a signal transduction molecule to 1175-tyrosine phosphorylated KDR/Flk-1.

3. A method for inhibiting angiogenesis, which comprises using a substance which inhibits binding of a signal transduction molecule to 1175-tyrosine phosphorylated KDR/Flk-1.

4. A method for screening a cell growth inhibitor, which comprises using a substance which inhibits binding of a signal transduction molecule to 1175-tyrosine phosphorylated KDR/Flk-1.

5. A method for screening an angiogenesis inhibitor, which comprises using a substance which inhibits binding of a signal transduction molecule to 1175-tyrosine phosphorylated KDR/Flk-1.

6. A method for screening a substance which inhibits KDR/Flk-1 signal transduction, which comprises using a substance which inhibits binding of a signal transduction molecule to 1175-tyrosine phosphorylated KDR/Flk-1.

7. A method for determining whether or not a tested substance can inhibit KDR/Flk-1 signal transduction, which comprises using a substance which inhibits binding of a signal transduction molecule to 1175-tyrosine phosphorylated KDR/Flk-1.

8. A method for detecting angiogenesis in tissue, which comprises using a substance which inhibits binding of a signal transduction molecule to 1175-tyrosine phosphorylated KDR/Flk-1.

9. A method for screening a substance which inhibits 1175-tyrosine phosphorylated KDR/Flk-1, which comprises using a substance which inhibits binding of a signal transduction molecule to 1175-tyrosine phosphorylated KDR/Flk-1.

10. The method according to any one of claims 1 to 9, wherein the signal transduction molecule is phospholipase C-γ (PLC-γ).

11. The method according to any one of claims 1 to 9, wherein the substance which inhibits binding of a signal transduction molecule to 1175-tyrosine phosphorylated KDR/Flk-1 is an antibody which specifically recognizes 1175-tyrosine phosphorylated KDR/Flk-1.

12. The method according to claim 11, wherein the antibody which specifically recognizes 1175-tyrosine phosphorylated KDR/Flk-1 is an antibody which binds to the 1175-tyrosine phosphorylated KDR/Flk-1 and inhibits phosphorylation of PLC-γ.

13. The method according to claim 11 or 12, wherein the antibody is a monoclonal antibody or the antibody fragment thereof.

14. A method for inhibiting KDR/Flk-1 signal transduction, which comprises using a substance which inhibits phosphorylation of 1175-tyrosine of KDR/Flk-1.

15. A method for inhibiting cell growth, which comprises using a substance which inhibits phosphorylation of 1175-tyrosine of KDR/Flk-1.

16. A method for inhibiting angiogenesis, which comprises using a substance which inhibits phosphorylation of 1175-tyrosine of KDR/Flk-1.

17. A method for determining whether or not a tested substance can inhibit KDR/Flk-1 signal transduction, which comprises using a substance which inhibits phosphorylation of 1175-tyrosine of KDR/Flk-1.

18. A method for detecting angiogenesis in tissue, which comprises using a substance which inhibits phosphorylation of 1175-tyrosine of KDR/Flk-1.

19. An agent comprising, as an active ingredient, a substance which inhibits binding of a signal transduction molecule to 1175-tyrosine phosphorylated KDR/Flk-1.

20. The agent according to claim 19, which is a tyrosine phosphorylation inhibitor.

21. The agent according to claim 19, which is a cell growth inhibitor.

22. The agent according to claim 19, which is an angiogenesis inhibitor.

23. The agent according to any one of claims 19 to 22, wherein the substance which inhibits binding of a signal transduction molecule to 1175-tyrosine phosphorylated KDR/Flk-1 is an antibody which specifically recognizes 1175-tyrosine phosphorylated KDR/Flk-1.

24. The agent according to claim 23, wherein the antibody which specifically recognizes 1175-tyrosine phosphorylated KDR/Flk-1 is an antibody which binds to the 1175-tyrosine phosphorylated KDR/Flk-1 and inhibits phosphorylation of phospholipase C-γ (PLC-γ).

25. The agent according to claim 23 or 24, wherein the antibody is a monoclonal antibody or an antibody fragment thereof.

26. A tyrosine phosphorylation inhibitor of KDR/Flk-1, comprising, as an active ingredient, a substance which inhibits phosphorylation of 1175-tyrosine of KDR/Flk-1.

27. A cell growth inhibitor, comprising as an active ingredient, a substance which inhibits phosphorylation of 1175-tyrosine of KDR/Flk-1.

28. An angiogenesis inhibitor, comprising, as an active ingredient, a substance which inhibits phosphorylation of 1175-tyrosine of KDR/Flk-1.

29. A compound which is obtained by the method according to any one of claims 4 to 6 and 9.

30. A monoclonal antibody or the antibody fragment thereof, which recognizes 1175-tyrosine phosphorylated KDR/Flk-1.

31. The monoclonal antibody or the antibody fragment thereof according to claim 30, which is a monoclonal antibody or the antibody fragment thereof which binds to 1175-tyrosine phosphorylated KDR/Flk-1 and inhibits binding of a signal transduction molecule to the phosphorylated KDR/Flk-1.

32. The monoclonal antibody or the antibody fragment thereof according to claim 33 or 34, which is a monoclonal antibody or the antibody fragment thereof which binds to 1175-tyrosine phosphorylated KDR/Flk-1 and inhibits phosphorylation of phospholipase C-γ (PLC-γ).

33. The monoclonal antibody or the antibody fragment thereof according to any one of claims 30 to 32, which is a monoclonal antibody which is produced by a hybridoma KM3035 (FERM BP-7729) or the antibody fragment thereof.

34. The antibody fragment according to any one of claims 30 to 33, wherein the antibody fragment is an antibody fragment selected from Fab, Fab', F(ab')₂, a single chain antibody (scFv), a dimerized variable region (V region) fragment (diabody), a disulfide stabilized V region fragment (dsFv) and a peptide comprising a complementarity determining region (CDR).

35. A monoclonal antibody or the antibody fragment thereof, in which the monoclonal antibody or the antibody fragment thereof according to any one of claims 30 to 34 is chemically or genetically conjugated with a radioisotope, a protein or an agent.

36. A DNA which encodes the monoclonal antibody or the antibody fragment thereof according to any one of claims 30 to 35.

37. A recombinant vector comprising the DNA according to claim 36.

38. A transformant comprising a host cell into which the recombinant vector according to claim 37 is introduced.


SEQUENCE LISTING

<110> KYOWA HAKKO KOGYO CO., LTD.

<110> SHIBUYA Masabumi

<120> Substance which inhibits biding of information transfer molecule
for 1175-tyrosine phosphorylated KDR/Flk-1 and usages of the same

<130> P-38125

<150> JP 2000-303694
<151> 2000-10-03

<160> 7

<170> PatentIn Ver. 2.1

<210> 1
<211> 11
<212> PRT
<213> Artificial Sequence

<220>
<221> Phosphorylation
<222> 6
<220>
<223> an antigen peptide for human KDR/Flk-1 phosphorylated at 1175-
tyrosine corresponding to its residue 1171-1180 and added cysteine
residue at the N-terminal

<400> 1
Cys Asp Gly Lys Asp Tyr Ile Val Leu Pro Ile

<210> 2
<211> 11
<212> PRT
<213> Artificial Sequence

<220>

<223> a peptide consisting of the same sequence as SEQ ID NO:1 without
phosphorylation
<400> 2
Cys Asp Gly Lys Asp Tyr Ile Val Leu Pro Ile


<210> 3
<211> 27
<212> DNA
<213> Artificial Sequence

<220>
<223> a primer for replacing of human KDR/Flk-1 tyrosine residue at
position 1175 to phenylalanine.

<400> 3
gatggcaaag acttcattgt tcttccc                                    27


<210> 4
<211> 27
<212> DNA
<213> Artificial Sequence

<220>
<223> a primer for replacing of human KDR/Flk-1 tyrosine residue at
position 1214 to phenylalanine.

<400> 4
cccaaattcc atttcgacaa cacagca                                    27


<210> 5
<211> 20
<212> DNA
<213> Artificial Sequence

<220>
<223> a primer for replacing of human KDR/Flk-1 tyrosine residue at
position 801 to phenylalanine.

<400> 5
gacaggcttc ttgtccatcg                                                    20

<210> 6
<211> 20
<212> PRT
<213> Human

<400> 6
Gln Gly Lys Asp Tyr Val Gly Ala Ile Pro Val Asp Leu Lys Arg Arg
1               5                   10                  15

Leu Asp Ser Ile
            20

<210> 7
<211> 1356
<212> PRT
<213> Human

<400> 7